(19) Europäisches Patentamt
European Patent Office
Office européen des brevets

(11) Veröffentlichungsnummer : **0 394 191 B1**

(12) **EUROPÄISCHE PATENTSCHRIFT**

(45) Veröffentlichungstag der Patentschrift : **02.02.94 Patentblatt 94/05**

(51) Int. Cl.$^5$ : **C07C 313/32,** A01N 47/24

(21) Anmeldenummer : **90810277.5**

(22) Anmeldetag : **05.04.90**

# (54) Sulfenylierte Carbaminsäureester.

Die Akte enthält technische Angaben, die nach dem Eingang der Anmeldung eingereicht wurden und die nicht in dieser Patentschrift enthalten sind.

(30) Priorität : **14.04.89 CH 1422/89**

(43) Veröffentlichungstag der Anmeldung : **24.10.90 Patentblatt 90/43**

(45) Bekanntmachung des Hinweises auf die Patenterteilung : **02.02.94 Patentblatt 94/05**

(84) Benannte Vertragsstaaten : **AT BE CH DE DK ES FR GB IT LI NL**

(56) Entgegenhaltungen :
**EP-A- 0 098 800**
**DE-A- 3 320 534**
**DE-A- 3 334 983**
**DE-A- 3 706 082**

(73) Patentinhaber : **CIBA-GEIGY AG**
**Klybeckstrasse 141**
**CH-4002 Basel (CH)**

(72) Erfinder : **Karrer, Friedrich, Dr.**
**Rebbergstrasse 5**
**CH-4800 Zofingen (CH)**

EP 0 394 191 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue substituierte 2-Phenoxyphenoxy-äthylcarbaminsäureester, ihre Herstellung, ihre Verwendung in der Schädlingsbekämpfung sowie Schädlingsbekämpfungsmittel, welche diese Carbaminsäureester als Wirkstoff enthalten.

Die erfindungsgemässen Carbaminsäureester entsprechen der Formel

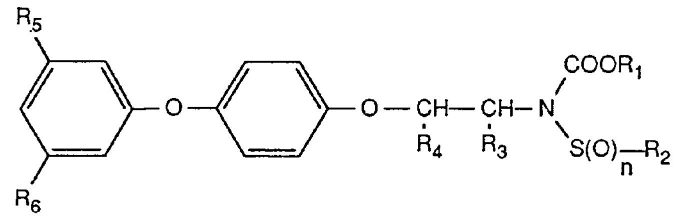

(I),

worin

$R_1$ $C_1$-$C_4$-Alkyl oder $C_3$-$C_4$-Alkenyl,

$R_2$ $C_1$-$C_4$-Alkyl oder einen Rest der Formel

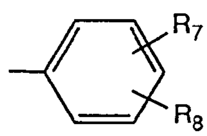

,

$R_3$ Wasserstoff oder Methyl, $R_4$ Wasserstoff,

$R_5$ und $R_5$ entweder beide Fluor oder $R_5$ Chlor und $R_5$ Wasserstoff,

$R_7$ und $R_5$ unabhängig voneinander Wasserstoff, Halogen oder $C_1$-$C_4$-Alkyl,

und

n null, eins oder zwei bedeuten.

Unter Halogen sind in der Definition von $R_7$ und $R_8$ Fluor, Chlor, Brom oder Jod zu verstehen, vorzugsweise aber Chlor.

$C_1$-$C_4$-Alkylgruppen können geradkettig oder verzweigt sein. Als Beispiele solcher Reste seien Methyl, Aethyl, Propyl, Isopropyl oder Butyl und seine Isomeren genannt. Die bevorzugte Alkylgruppe ist Aethyl. $C_3$-$C_4$-Alkenylgruppen tragen die Doppelbindung vorzugsweise in 2-Stellung, wie bei Allyl, Methallyl oder 2-Butenyl. Die bevorzugte Alkenylgruppe ist Allyl.

Pestizide Aethylcarbaminsäure-Derivate sind aus der Literatur schon verschiedentlich bekannt geworden, jedoch befriedigen diese Substanzen bezüglich des erzielten Wirkungsspektrums nicht oder nur teilweise. Solche Verbindungen sind beispielsweise bekannt aus den US-Patenten 4 080 470, 4 215 139, 4 413 010, 4 555 405, 4 608 389 und 4 745 128 sowie den Deutschen Offenlegungsschriften DE-OS 3 320 534, 3 334 983 und 3 706 082. Es besteht somit noch immer ein Bedürfnis nach Wirkstoffen dieser Substanzklasse mit verbesserten Eigenschaften.

Es wurde nun gefunden, dass die erfindungsgemässen Verbindungen der Formel I bei günstiger Warmblüter-, Fisch- und Pflanzenverträglichkeit wertvolle Wirkstoffe in der Schädlingsbekämpfung sind. Insbesondere betrifft die Anwendung der erfindungsgemässen Wirkstoffe Insekten und Spinnentiere, die in Nutz- und Zierpflanzen in der Landwirtschaft, insbesondere in Baumwoll-, Gemüse- und Obstpflanzungen, im Forst, im Vorrats- und Materialschutz sowie im Hygienesektor insbesondere an Haus- und Nutztieren vorkommen. Sie sind gegen alle oder einzelne Entwicklungsstadien von normal sensiblen aber auch resistenten Arten wirksam. Dabei kann sich ihre Wirkung in unmittelbarer Abtötung der Schädlinge oder erst nach einiger Zeit, beispielsweise bei einer Häutung, oder in einer verminderten Eiablage und/oder Schlupfrate zeigen. Zu den oben erwahnten Schädlingen gehören:

aus der Ordnung Lepidoptera zum Beispiel

Acleris spp., Adoxophyes spp., Aegeria spp., Agrotis spp., Alabama argillaceae, Amylois spp., Anticarsia gemmatalis, Archips spp., Argyrotaenia spp., Autographa spp., Busseola fusca, Cadra cautella, Carposina

nipponensis, Chilo spp., Choristoneura spp., Clysia ambiguella, Cnaphalocrocis spp., Cnephasia spp., Cochylis spp., Coleophora spp., Crocidolomia binotalis, Cryptophlebia leucotreta, Cydia spp., Diatraea spp., Diparopsis castanea, Earias spp., Ephestia spp., Eucosma spp., Eupoecilia ambiguella, Euproctis spp., Euxoa spp., Grapholita spp., Hedya nubiferana, Heliothis spp., Hellula undalis, Hyphantria cunea, Keiferia lycopersicella, Leucoptera scitella, Lithocollethis spp., Lobesia botrana, Lymantria spp., Lyonetia spp., Malacosoma spp., Mamestra brassicae, Manduca sexta, Operophtera spp., Ostrinia nubilalis, Pammene spp., Pandemis spp., Panolis flammea, Pectinophora gossypiella, Phthorimaea operculella, Pieris rapae, Pieris spp., Plutella xylostella, Prays spp., Scirpophaga spp., Sesamia spp., Sparganothis spp., Spodoptera spp., Synanthedon spp., Thaumetopoea spp., Tortrix spp., Trichoplusia ni und Yponomeuta spp.;
aus der Ordnung Coleoptera zum Beispiel
Agriotes spp., Anthonomus spp., Atomaria linearis, Chaetocnema tibialis, Cosmopolites spp., Curculio spp., Dermestes spp., Diabrotica spp., Epilachna spp., Eremnus spp., Leptinotarsa decemlineata, Lissorhoptrus spp. Melolontha spp., Orycaephilus spp., Otiorhynchus spp., Phlyctinus spp., Popillia spp., Psylliodes spp., Rhizopertha spp., Scarabeidae, Sitophilus spp., Sitotroga spp., Tenebrio spp., Tribolium spp. und Trogoderma spp.;
aus der Ordnung der Orthoptera zum Beispiel
Blatta spp., Blattella spp., Gryllotalpa spp., Leucophaea maderae, Locusta spp., Periplaneta spp. und Schistocerca spp.;
aus der Ordnung der Isoptera zum Beispiel
Reticulitermes spp.;
aus der Ordnung der Psocoptera zum Beispiel
Liposcelis spp.;
aus der Ordnung der Anoplura zum Beispiel
Haematopinus spp., Linognathus spp., Pediculus spp., Pemphigus spp. und Phylloxera spp.;
aus der Ordnung der Mallophaga zum Beispiel
Damalinea spp. und Trichodectes spp.;
aus der Ordnung der Thysanoptera zum Beispiel
Frankliniella spp., Hercinothrips spp., Taeniothrips spp., Thrips palmi, Thrips tabaci und Scirtothrips aurantii;
aus der Ordnung der Heteroptera zum Beispiel
Cimex spp., Distantiella theobroma, Dysdercus spp., Euchistus spp., Eurygaster spp., Leptocorisa spp., Nezara spp., Piesma spp., Rhodnius spp., Sahlbergella singularis, Scotinophara spp. und Triatoma spp.;
aus der Ordnung der Homoptera zum Beispiel
Aleurothrixus floccosus, Aleyrodes brassicae, Aonidiella spp, Aphididae, Aphis spp., Aspidiotus spp., Bemisia tabaci, Ceroplaster spp., Chrysomphalus aonidium, Chrysomphalus dictyospermi, Coccus hesperidum, Empoasca spp., Eriosoma larigerum, Erythroneura spp., Gascardia spp., Laodelphax spp., Lecanium corni, Lepidosaphes spp., Macrosiphus spp., Myzus spp., Nephotettix spp., Nilaparvata spp., Paratoria spp., Pemphigus spp., Planococcus spp., Pseudaulacaspis spp., Pseudococcus spp., Psylla spp., Pulvinaria aethiopica, Quadraspidiotus spp., Rhopalosiphum spp., Saissetia spp., Scaphoideus spp., Schizaphis spp., Sitobion spp., Trialeurodes vaporariorum, Trioza erytreae und Unaspis citri;
aus der Ordnung der Hymenoptera zum Beispiel
Acromyrmex, Atta spp., Cephus spp., Diprion spp., Diprionidae, Gilpinia polytoma, Hoplocampa spp., Lasius spp., Monomorium pharaonis, Neodiprion spp., Solenopsis spp. und Vespa spp.;
aus der Ordnung der Diptera zum Beispiel
Aedes spp., Antherigona soccata, Bibio hortulanus, Calliphora erythrocephala, Ceratitis spp., Chrysomyia spp., Culex spp., Cuterebra spp., Dacus spp., Drosophila melanogaster, Fannia spp., Gastrophilus spp., Glossina spp., Hypoderma spp., Hyppobosca spp., Liriomyza spp., Lucilia spp., Melanagromyza spp., Musca spp., Oestrus spp., Orseolia spp., Oscinella frit, Pegomyia hyoscyami, Phorbia spp., Rhagoletis pomonella, Sciara spp., Stomoxys spp., Tabanus spp., Tannia spp. und Tipula spp.;
aus der Ordnung der Siphonaptera z.B.
Ceratophyllus spp., Xenopsylla cheopis,
aus der Ordnung der Acarina zum Beispiel
Acarus siro, Aceria sheldoni, Aculus schlechtendali, Amblyomma spp., Argas spp., Boophilus spp., Brevipalpus spp., Bryobia praetiosa, Calipitrimerus spp., Chorioptes spp., Dermanyssus gallinae, Eotetranychus carpini, Eriophyes spp., Hyalomma spp., Ixodes spp., Olygonychus pratensis, Omithodoros spp., Panonychus spp., Phyllocoptruta oleivora, Polyphagotarsonemus latus, Psoroptes spp., Rhipicephalus spp., Rhizoglyphus spp., Sarcoptes spp., Tarsonemus spp. und Tetranychus spp.;
und
aus der Ordnung der Thysanura zum Beispiel

Lepisma saccharina.

Von besonderer Bedeutung hat sich bei den erfindungsgemässen Verbindungen die Verwendung bei der Bekämpfung von Reiszikaden zum Beispiel aus den Familien Delphacidae und Cicadellidae, wie Nilaparvata lugens, Laodelphax striatellus und Nephotettix cincticeps erweisen. Ebenfalls herausragende Wirkung zeigen die Wirkstoffe der Formel I gegen die schwierig unter Kontrolle zu bringenden sogenannten "Weissen Fliegen" der Familie Aleyrodidae, Gattungen Bemisia und Trialeurodes wie Bemisia tabaci oder Trialeurodes vaporarium. Sehr gute Wirkungen erzielen die Verbindungen der Formel I gegen Obstbaumschädlinge der Familie Tortricidae und Olethreutidae mit den Gattungen Cydia, Adoxophyes und Lobesia, wie z.B. Cydia pomonella, Adoxophyes orana und Lobesia botrana.

Im wesentlichen bewirken die Verbindungen der Formel I in den Zielgruppen von Schädlingen eine Wachstumshemmung bei den verschiedenen Entwicklungsstufen, so dass die Verminderung des Schädlingsbefalls auf Entwicklungsstörungen der Schädlinge, insbesondere einen chemosterilisierenden und oviziden Effekt zurückzuführen ist.

Wegen ihrer vorteilhaften Wirkung sind unter den Verbindungen der Formel I folgende Einzelverbindungen hervorzuheben:

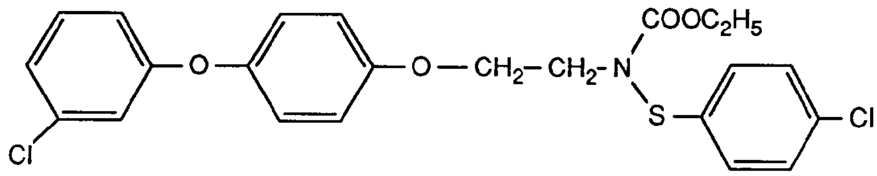
,

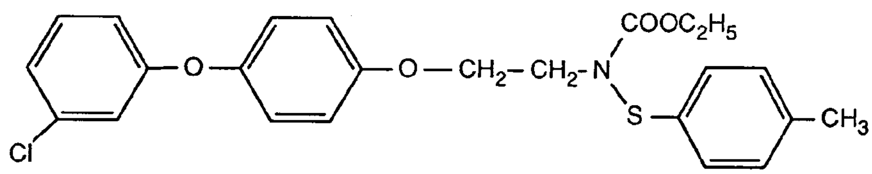
,

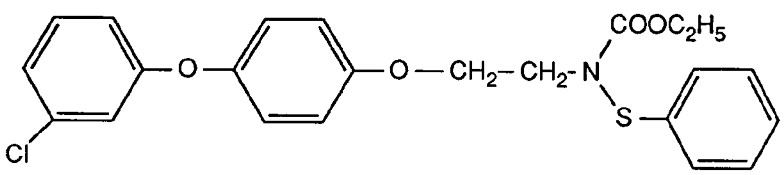
,

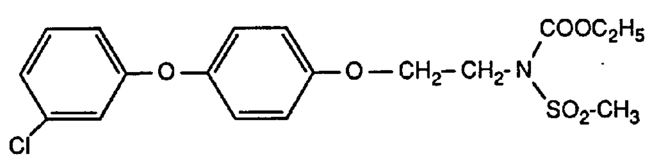
,

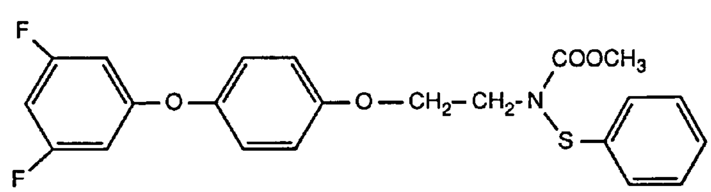
,

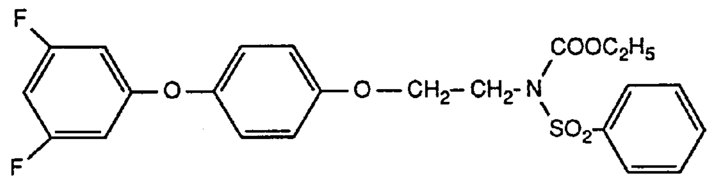

,

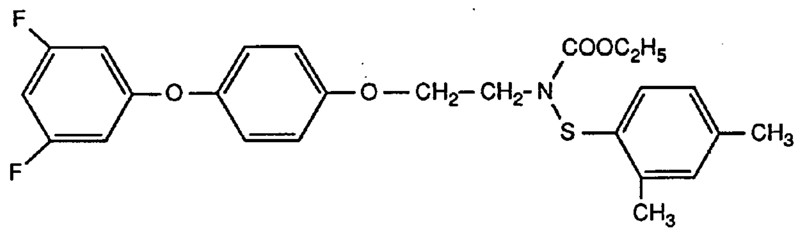

,

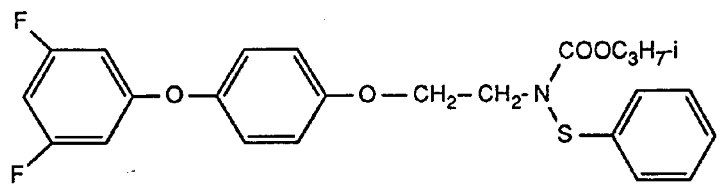

,

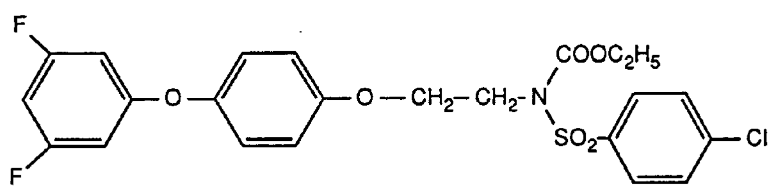

,

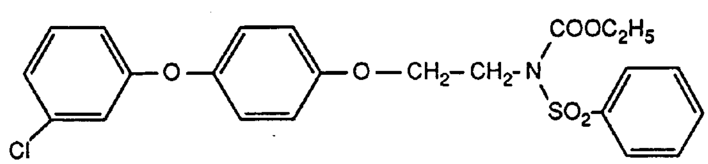

,

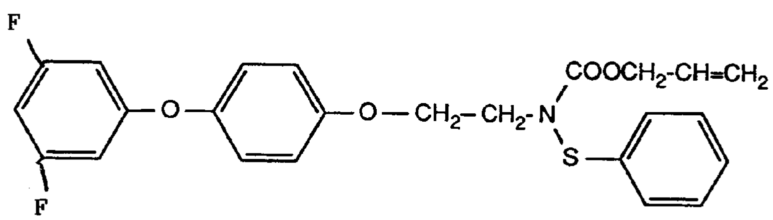

,

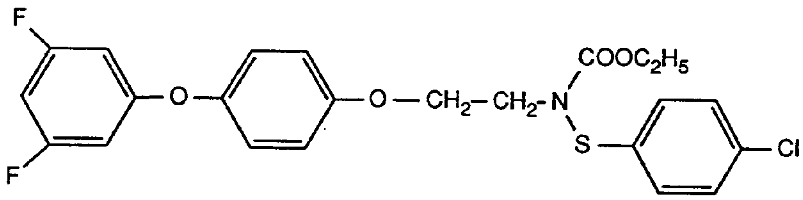

,

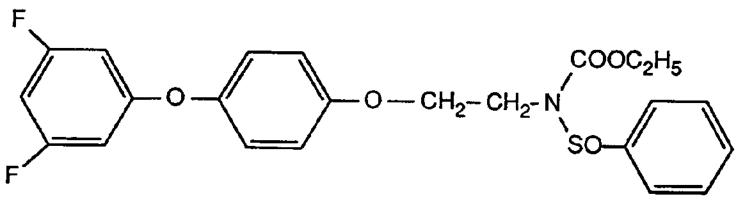

,

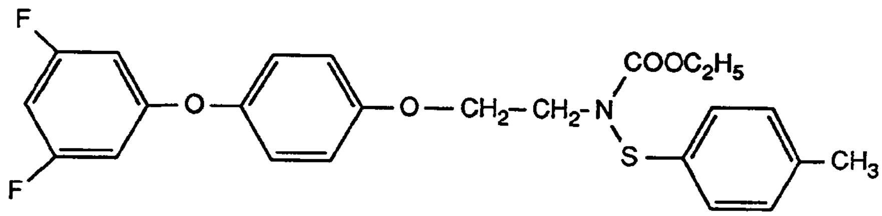

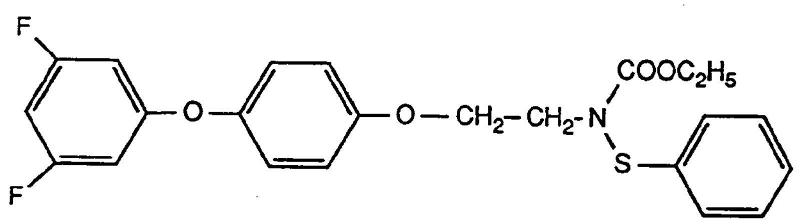

und

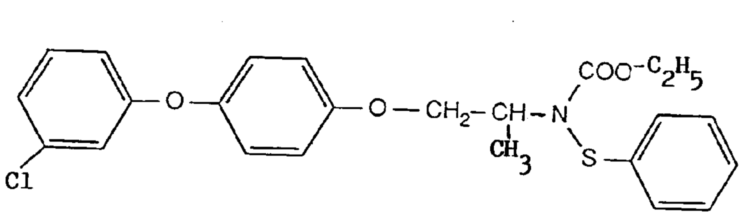

.

Die erfindungsgemässen Verbindungen der Formel I können nach an sich bekannten Methoden hergestellt werden. So kann man die Verbindungen der Formel I beispielsweise erhalten, indem man entweder

a) ein Phenoxyphenol der Formel II

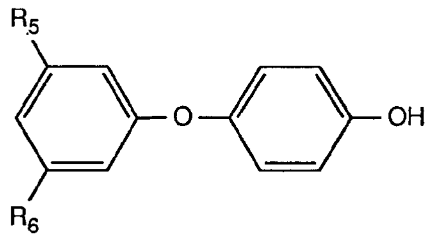

(II),

worin $R_5$ und $R_6$ die unter Formel I gegebenen Bedeutungen haben, in Gegenwart einer Base mit einem Aethylcarbaminsäure-Derivat der Formel

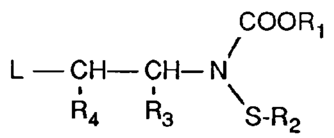

(III),

worin $R_1$, $R_2$, $R_3$ und $R_4$ die unter Formel I gegebenen Bedeutungen haben und L für eine Abgangsgruppe, wie zum Beispiel Halogen, vorzugsweise Chlor oder Brom, oder eine Sulfonyloxygruppe, wie zum Beispiel Methylsulfonyloxy, Trifluormethylsulfonyloxy, Phenylsulfonyloxy oder Tolylsulfonyloxy stehen, umsetzt und das erhaltene Produkt der Formel

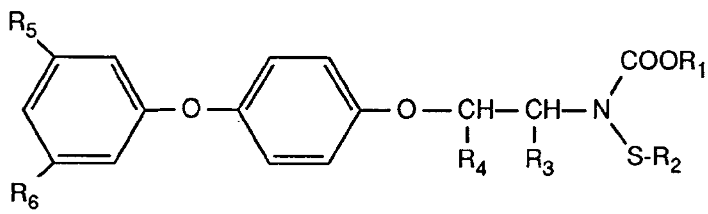

(Ia),

worin $R_1$, $R_2$, $R_3$, $R_4$, $R_5$ und $R_5$ die unter Formel I gegebenen Bedeutungen haben, gewünschtenfalls zu einer Verbindung der Formel I, worin n eins oder zwei bedeutet, oxidiert; oder

b) ein Aethylcarbaminsäure-Derivat der Formel

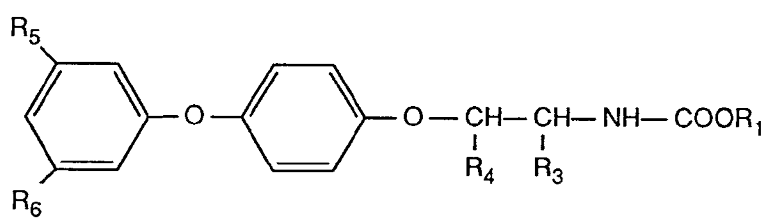

(IV),

worin $R_1$, $R_3$, $R_4$, $R_5$ und $R_6$ die unter Formel I gegebenen Bedeutungen haben, in Gegenwart einer Base mit einem Sulfensäurehalogenid der Formel

$$Hal\text{-}S\text{-}R_2 \qquad (V),$$

worin $R_2$ die unter Formel I gegebene Bedeutung hat, und Hal für Halogen, vorzugsweise Chlor steht, umsetzt und das erhaltene Produkt der Formel Ia gewünschtenfalls zu einer Verbindung der Formel I, worin n eins oder zwei bedeutet, oxidiert; oder

c) ein Aethylcarbaminsäure-Derivat der Formel IV in Gegenwart einer Base mit einem Sulfinsäurehalogenid der Formel

$$Hal\text{-}SO\text{-}R_2 \qquad (VI),$$

worin $R_2$ die unter Formel I gegebene Bedeutung hat, und Hal für Halogen, vorzugsweise Chlor steht, umsetzt und das erhaltene Produkt der Formel

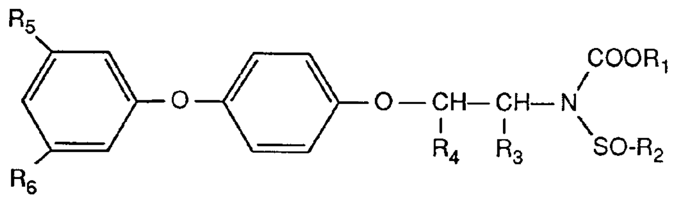

(Ib),

worin $R_1$, $R_2$, $R_3$, $R_4$, $R_5$ und $R_5$ die unter Formel I gegebenen Bedeutungen haben, gewünschtenfalls zu einer Verbindung der Formel I, worin n zwei bedeutet, oxidiert; oder

d) ein Aethylcarbaminsäure-Derivat der Formel IV in Gegenwart einer Base mit einem Sulfonsäurehalogenid der Formel VII

$$Hal\text{-}SO_2\text{-}R_2 \quad (VII),$$

worin $R_2$ die unter Formel I gegebene Bedeutung hat und Hal für Halogen, vorzugsweise Chlor steht, zu einer Verbindung der Formel

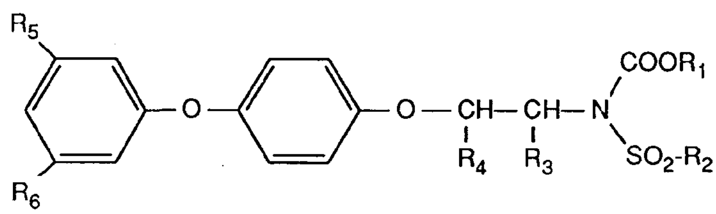

(Ic),

worin $R_1$, $R_2$, $R_3$, $R_4$, $R_5$ und $R_5$ die unter Formel I gegebenen Bedeutungen haben umsetzt; oder

e) ein Aethylaminderivat der Formel

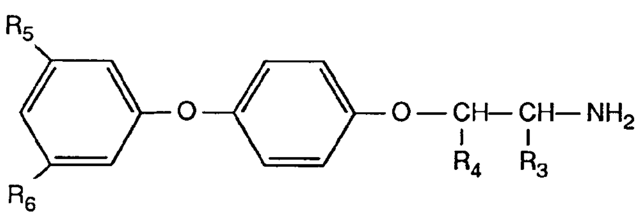

(VIII),

worin $R_3$, $R_4$, $R_5$ und $R_5$ die unter Formel I gegebenen Bedeutungen haben, in Gegenwart einer Base mit einem Halogenameisensäurederivat der Formel IX

$$Hal\text{-}COOR_1 \quad (IX),$$

worin $R_1$ die unter Formel I gegebene Bedeutung hat und Hal für Halogen, vorzugsweise Chlor steht, acyliert und das erhaltene Zwischenprodukt der Formel IV in Gegenwart einer Base mit einem Sulfensäurehalogenid der Formel V sulfenyliert und das erhaltene Produkt der Formel Ia gewünschtenfalls zu einer Verbindung der Formel I, worin n eins oder zwei bedeutet, oxidiert; oder

f) ein Aethylaminderivat der Formel VIII in Gegenwart einer Base mit einem Sulfensäurehalogenid der Formel V sulfeniert und das erhaltene Zwischenprodukt der Formel

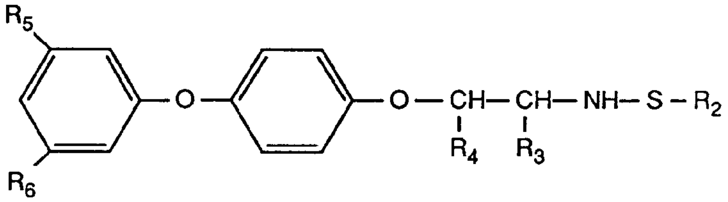

(X),

worin $R_2$, $R_3$, $R_4$, $R_5$ und $R_6$ die unter Formel I gegebenen Bedeutungen haben, in Gegenwart einer Base mit einem Halogenameisensäurederivat der Formel IX acyliert und das erhaltene Produkt der Formel Ia gewünschtenfalls zu einer Verbindung der Formel I, worin n eins oder zwei bedeutet, oxidiert; oder
g) ein Alkalisalz des Sulfonamids der Formel Xa mit einem Halogenameisensäurederivat der Formel IX umsetzt, wobei $R_1$ bis $R_6$ die unter Formel I angegebenen Bedeutungen haben und $Me^{\oplus}$ ein Alkalimetallkation bedeutet:

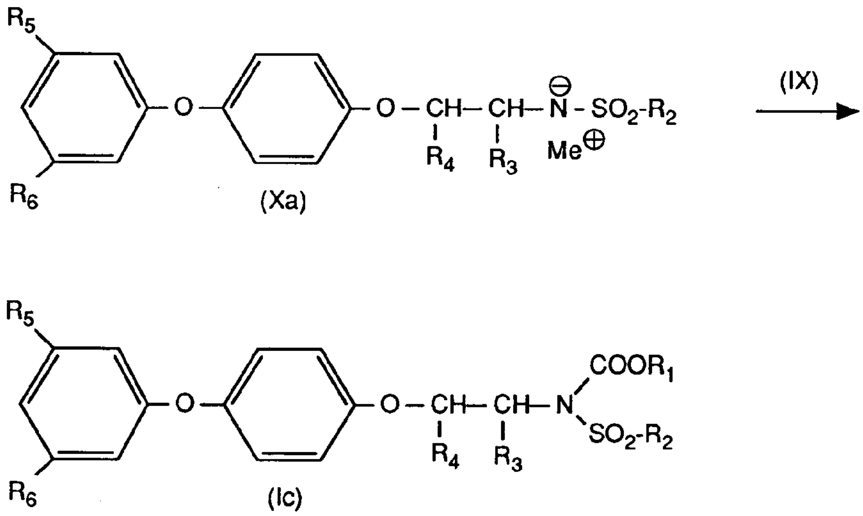


(Xa)

(Ic)

Die erwähnten Verfahren a), b), c), d), e), f) und g) können vorzugsweise unter normalem Druck und in Gegenwart eines inerten organischen Lösungs- oder Verdünnungsmittels durchgeführt werden. Als Lösungs- oder Verdünnungsmittel eignen sich je nach Reaktionstyp beispielsweise Aether und ätherartige Verbindungen, wie Dipropyläther, Dibutyläther, Dioxan, Dimethoxyäthan und Tetrahydrofuran; N,N-dialkylierte Carbonsäureamide, wie N,N-Dimethylformamid, aliphatische, aromatische sowie halogenierte Kohlenwasserstoffe, insbesondere Benzol, Toluol, Xylol, Chloroform, Methylenchlorid, Tetrachlorkohlenstoff und Chlorbenzol; Dimethylsulfoxid, Sulfolan sowie Ketone, zum Beispiel Aceton, Methyläthylketon, Methylisopropylketon und Methylisobutylketon.

Die Reaktionstemperaturen bei diesen Verfahren liegen im allgemeinen zwischen -20°C und dem Siedepunkt des Reaktionsgemisches. Vorzugsweise wird eine obere Temperatur von +100°C nicht überschritten. Bevorzugt sind Temperaturbereiche zwischen 0° und +50°C, insbesondere aber zwischen 0° und +40°C.

Als Basen eignen sich im Verfahren a) Alkali- oder Erdalkalihydride wie Lithiumhydrid, Natriumhydrid oder Calciumhydrid; Alkalihydroxide wie Natrium- oder Kaliumhydroxid; Alkalicarbonate wie Natrium- oder Kaliumcarbonat; Alkoholate wie Natrium- oder Kaliumäthylat oder -methylat oder Kalium-tert.butylat. Ebenfalls eignen sich tertiäre Amine als Basen wie beispielsweise Triäthylamin, Pyridin oder Diisopropyläthylamin. Die Verätherungsreaktion lässt sich dann unter milderen Bedingungen durchführen, wenn die Abgangsgruppe L aus der Reihe der Sulfonsäureabkömmlinge wie Methansulfonsäure oder Toluolsulfonsäure ausgewählt wird, oder wenn man für den Fall dass L für Fluor, Chlor oder Brom steht, eine katalytische Menge Alkalijodid, z.B. Kaliumjodid, zusetzt.

Als Basen in den Verfahren b), c), d), e) und f) eignen sich vorzugsweise tertiäre Amine wie Trialkylamine, Pyridine oder N,N-Dialkylaniline, beispielsweise Triäthylamin, Diisopropyläthylamin, Pyridin, 4-Dimethylaminopyridin, Dimethylanilin oder Diäthylanilin.

Im Falle von flüssigen tertiären Aminen können diese gleichzeitig als Lösungsmittel für die Reaktion eingesetzt werden.

Wird eine Aenderung der Oxidationsstufe der Schwefelatome in den nach Verfahren a), b), c), d), e) oder f) erhaltenen Verbindungen gewünscht, so kann dies durch an sich bekannte Oxidationsreaktionen erfolgen. Als Reagenzien kommen übliche Oxidationsmittel unter üblichen Reaktionsbedingungen in Frage. Als Oxidationsmittel seien beispielsweise genannt: Persäuren, wie Peressigsäure, Perameisensäure, Perbenzoesäure, 3-Chlorperbenzoesäure oder Monoperphthalsäure, Wasserstoffperoxid, Perjodsäure, Natriumperjodat $NaJO_4$, sowie Alkalichromate. Während organische Persäuren in typischen Reaktionen in chlorierten Kohlenwasserstoffen wie Methylenchlorid, Chloroform, Aethylenchlorid umgesetzt werden, kommen für anorganische Oxidationsmittel wasserhaltige und/oder mit Wasser mischbare Lösungsmittel wie Aceton, Essigsäure oder Ameisensäure in Frage. Vorzugsweise wird Perameisensäure in situ hergestellt, beispielsweise durch Zusatz von $H_2O_2$ zu Ameisensäure. Reaktionstemperaturen der Oxidationsgemische liegen zwischen -20°C und +60°C.

Die Zwischenprodukte der Formeln II, IV, V, VI, VII und IX sind bekannt oder können nach an sich bekannte Methoden hergestellt werden.

Die Zwischenprodukte der Formel III können alternativ nach den folgenden Schemata erhalten werden:

Schema 1:

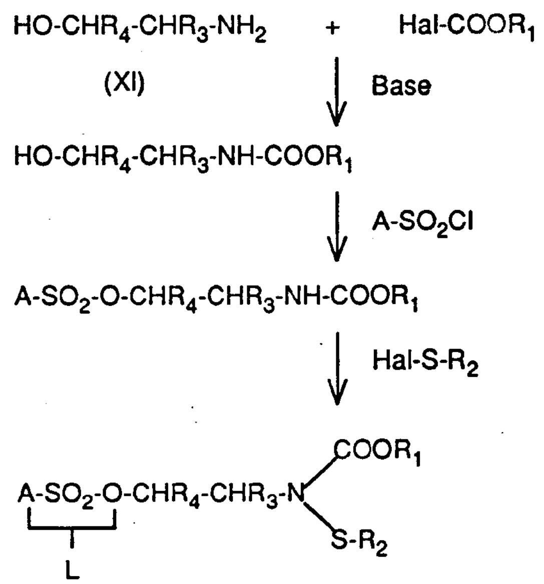


(IX)

(XI)

(XII)

(XIII)

(IIIa)

| | |
|---|---|
| Hal: | Halogen, vorzugsweise Chlor, |
| A: | organischer Rest, vorzugsweise $CH_3$, $CF_3$, Phenyl, Tolyl, |
| L: | Abgangsgruppe, |
| $R_1$, $R_2$, $R_3$, $R_4$, Y, Z: | definiert wie unter Formel I, |

oder

Schema 2:

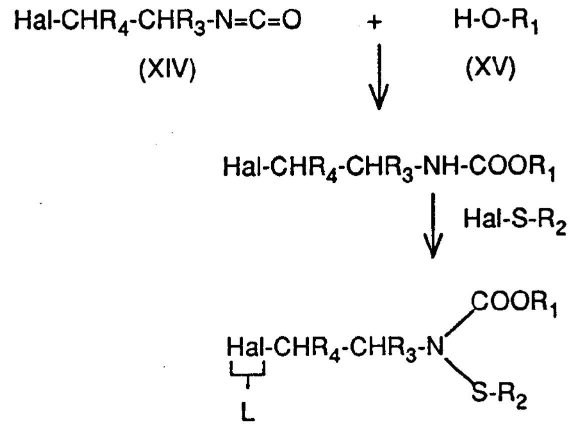


(XIV) (XV)

(XVI)

(IIIb)

Hal: Halogen, vorzugsweise Chlor,
A: organischer Rest, vorzugsweise $CH_3$, $CF_3$, Phenyl, Tolyl,
L: Abgangsgruppe,
$R_1$, $R_2$, $R_3$, $R_4$: definiert wie unter Formel I.

Die Zwischenprodukte der Formel VIII können durch "reduktive Aminierung" aus den entsprechenden Carbonylverbindungen hergestellt werden:

Schema 3:

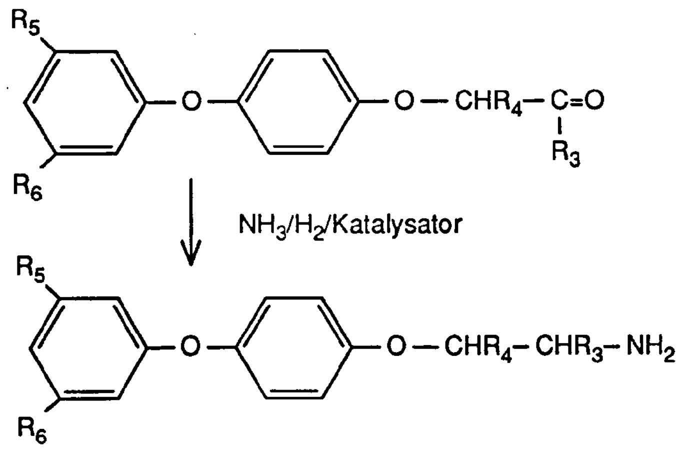


(XVII)

(VIII)

Katalysator: übliche Hydrierungskatalysatoren, wie Palladium, Platin, insbesondere Raney-Nickel,
$R_3$, $R_4$, $R_5$, $R_6$: definiert wie unter Formel I.

Anstelle des Reduktionssystems $H_2$/Katalysator können nach Bildung des Imins aus XVII mit Ammoniak auch andere reduzierende Reagenzien verwendet werden, wie komplexe Hydride, z.B. Natriumborhydrid.

Die in den Schemata 1,2 und 3 eingesetzten Reagenzien sind meist bekannt, im Handel erhältlich oder sie lassen sich durch einfache Methoden aus bekannten Produkten herstellen. Die Reaktionsbedingungen entsprechen denjenigen der bekannten analogen Reaktionen.

Die Wirkung der erfindungsgemässen Verbindungen, beziehungsweise der sie enthaltenden Mittel, lässt sich durch Zusatz von anderen Insektiziden und/oder Akariziden wesentlich verbreitern und an gegebene Umstände anpassen. Als solche Zusätze kommen zum Beispiel organische Phosphorverbindungen, Nitrophenole und deren Derivate, Formamidine, Harnstoffe, Carbamate, Pyrethroide, chlorierte Kohlenwasserstoffe und Bacillus thuringiensis-Präparate in Betracht.

Mit besonderem Vorteil kann man die Verbindungen der Formel I auch mit Substanzen kombinieren, welche einen pestizid verstärkenden Effekt ausüben. Beispiele solcher Verbindungen sind unter anderem: Piperonylbutoxid, Propinyläther, Propinyloxime, Propinylcarbamate und Propinylphosphonate, 2-(3,4-Methylendioxyphenoxy)-3,6,9-trioxaundecan oder S,S,S-Tributylphosphorotrithioate.

Die Verbindungen der Formel I werden in unveränderter Form oder vorzugsweise zusammen mit den in der Formulierungstechnik üblichen Hilfsmitteln eingesetzt und werden daher beispielsweise zu Emulsionskonzentraten, direkt versprühbaren oder verdünnbaren Lösungen, verdünnten Emulsionen, Spritzpulvern, löslichen Pulvern, Stäubemitteln, Granulaten oder auch Verkapselungen in zum Beispiel polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierungen, das heisst die den Wirkstoff der Formel I oder Kombinationen dieser Wirkstoffe mit andern Insektiziden oder Akariziden, und gegebenenfalls einen festen oder flüssigen Zusatzstoff enthaltenden Mittel, Zubereitungen oder Zusammensetzungen, werden in bekannter Weise hergestellt, wie durch inniges Vermischen und/oder Vermahlen der Wirkstoffe mit Streckmitteln, wie zum Beispiel mit Lösungmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$-$C_{12}$, wie zum Beispiel Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethlenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxydierte Pflanzenöle, wie epoxydiertes Kokosnussöl oder Sojaöl, oder Wasser.

Als feste Trägerstoffe, beispielsweise für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden.

Als gekörnte, adsorptive Granulatträger kommen sowohl poröse Typen, wie zum Beispiel Bimsstein, Ziegelbruch, Sepiolit oder Bentonit, als auch nicht sorptive Trägermaterialien wie Calcit oder Sand in Frage. Darüberhinaus kann eine Vielzahl von vorgranulierten Materialien anorganischer oder organischer Natur, wie insbesondere Dolomit oder zerkleinerte Pflanzenrückstände, verwendet werden.

Als oberflächenaktive Verbindungen kommen je nach der Art des zu formulierenden Wirkstoffes der Formel I oder der Kombinationen dieser Wirkstoffe und andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sogenannte wasserlösliche Seifen wie auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie die Natrium oder Kalium-Salze der Oel- oder Stearinsäure, oder von natürlichen Fettsäuregemischen, die beispielsweise aus Kokosnuss- oder Talgöl gewonnen werden können. Ferner sind auch die Fettsäuremethyltaurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, zum Beispiel das Na- oder Calcium-Salz der Ligninsulfonsäure, des Dodecylschwefelsäureesters oder eines aus natürlichen Fettsäuren hergestellten Fettalkoholsulfatgemisches. Hierher gehären auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8 - 22 C-Atomen. Arylalkylsulfonate sind beispielsweise die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie die Salze des Phosphorsäureesters eines p-Nonylphenol-(4-14)-Aethylenoxid-Adduktes in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykoleinheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, zum Beispiel das Stearyltrimethylammoniumchlorid oder das Benzyl-di-(2-chloräthyl)-äthylammoniumbromid.

Die in der Formulierungstechnik gebräuchlichen Tenside sind unter anderem in den folgenden Publikationen beschrieben:
"1985 International Mc Cutcheon's Emulsifiers &Detergents", Glen Rock NJ USA, 1985",
H. Stache, "Tensid-Taschenbuch", 2. Aufl., C. Hanser Verlag München, Wien 1981,
M. and J. Ash. "Encyclopedia of Surfactants", Vol. I-III, Chemical Publishing Co., New York, 1980-1981.

Die pestiziden Zubereitungen enthalten in der Regel 0,1 bis 99 %, insbesondere 0,1 bis 95 %, des Wirkstoffs der Formel I oder Kombinationen dieses Wirkstoffs mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen. Typische Anwendungskonzentrationen liegen zwischen 0,1 und 1000 ppm, vorzugsweise zwischen 0,1 und 500 ppm. Die Aufwandmengen pro Hektar betragen im allgemeinen 10 bis 1000 g Wirkstoff pro Hektar, vorzugsweise 25 bis 250 g/ha.

Insbesondere setzen sich bevorzugte Formulierungen folgendermassen zusammen:
(%=Gewichtsprozent)

Emulgierbare Konzentrate

| | |
|---|---|
| Aktiver Wirkstoff: | 1 bis 20 %, bevorzugt 5 bis 10 % |
| oberflächenaktives Mittel: | 5 bis 30 %, vorzugsweise 10 bis 20 % |
| flüssiges Trägermittel: | 50 bis 94 %, vorzugsweise 70 bis 85 % |

Stäube:

| | |
|---|---|
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |

Suspensions-Konzentrate

| | |
|---|---|
| Aktiver Wirkstoff: | 5 bis 75 %, vorzugsweise 10 bis 50 % |
| Wasser: | 94 bis 24 %, vorzugsweise 88 bis 30 % |
| oberflächenaktives Mittel: | 1 bis 40 %, vorzugsweise 2 bis 30 % |

Benetzbare Pulver

| | |
|---|---|
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermaterial: | 5 bis 95 %, vorzugsweise 15 bis 90 % |

Granulate

| | |
|---|---|
| Aktiver Wirkstoff: | 0,5 bis 30 %, vorzugsweise 3 bis 15 % |
| festes Trägermittel: | 99,5 bis 70 %, vorzugsweise 97 bis 85 % |

Die Mittel können auch weitere Zusätze wie Stabilisatoren, Entschäumer, Konservierungsmittel, Viskositätsregulatoren, Bindemittel, Haftmittel sowie Dünger oder andere Wirkstoffe zur Erzielung spezieller Effekte enthalten.

Die folgenden Beispiele dienen der Erläuterung der Erfindung. Sie schränken die Erfindung nicht ein.

Beispiel H1: N-Phenylthio-2-[4-(3,5-difluorphenoxy)-phenoxy]-äthylcarbaminsäure-äthylester

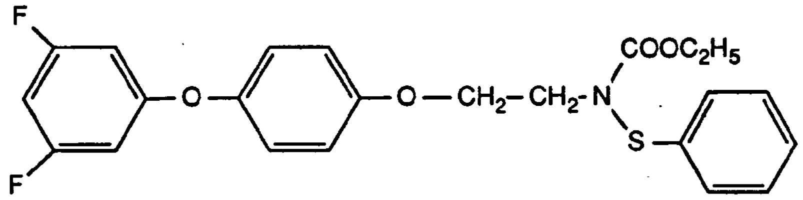

a) Zu der Lösung von 22,2g 4-(3,5-Difluorphenoxy)-phenol in 80 ml Dimethylformamid werden 27,6 g fein gepulvertes Kaliumcarbonat und 1,0 g pulverisiertes Kaliumjodid zugesetzt. Ferner lässt man 18,2 g 2-Chloräthylcarbaminsäure-äthylester zutropfen und erwärmt das Reaktionsgemisch unter Rühren für 16 Stunden auf +95°C. Hierauf wird das Reaktionsgemisch filtriert, das Filtrat in 400 ml Wasser gegossen und dreimal mit Diäthyläther extrahiert. Die vereinigten organischen Phasen werden mit Wasser gewaschen über Natriumsulfat getrocknet und das Lösungsmittel abdestilliert. Der Rückstand wird über Kieselgel (Eluierungsmittel: n-Hexan/Diäthyläther, 3:1) gereinigt, wodurch der reine 2-[4(3,5-Difluorphenoxy)phenoxy]-äthylcarbaminsäure-äthylester als farbloser, kristallin erstarrender Feststoff vom Smp. 54-56°C erhalten wird, Ausbeute: 92 % der Theorie.

b) Zu einer Lösung von 12 g 2-[4-(3,5-Difluorphenoxy)-phenoxy]-äthylcarbaminsäure-äthylester in 40 ml Pyridin lässt man unter Rühren bei 0° bis +5°C innerhalb von 30 Minuten 5,7 g frisch destilliertes Phenylsulfenylchlorid in 10 ml Toluol zutropfen. Anschliessend wird weitere 4 Stunden bei Raumtemperatur gerührt. Das Reaktionsgemisch wird auf 300 ml 2N-Salzsäure und Eis gegossen und zweimal mit Diäthyläther extrahiert. Die vereinigten organischen Phasen werden mit Wasser und Natriumchloridlösung neutral gewaschen und über Natriumsulfat getrocknet. Das Lösungsmittel wird im Vakuum abdestilliert und der Rückstand an Kieselgel chromatographisch gereinigt (Eluierungsmittel: Hexan/Diäthyläther, 20:1), wodurch reiner N-Phenylthio-2-[4-(3,5-difluorphenoxy)-phenoxy]-äthylcarbaminsäure-äthylester erhalten wird, $n_D^{20}$: 1,5700.

Beispiel H2: N-(4-Chlorphenylthio)-2-[4-(3,5-difluorphenoxy)-phenoxy]-äthylcarbaminsäure-äthylester

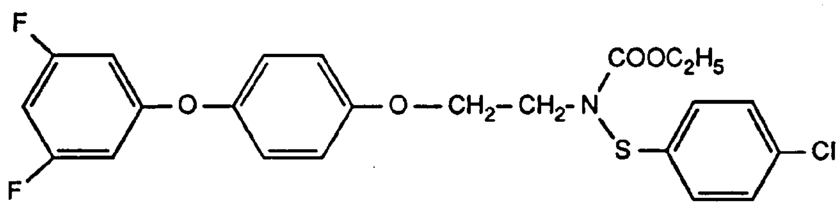

Zu einer Lösung von 12 g 2-[4-(3,5-Difluorphenoxy)phenoxy]-äthylcarbaminsäure-äthylester in 40 ml Pyridin lässt man unter Rühren bei 0° bis +5°C innerhalb von 30 Minuten 7,6 g frisch destilliertes 4-Chlorphenylsulfenylchlorid zutropfen. Anschliessend wird weitere 16 Stunden bei Raumtemperatur gerührt. Das Reaktionsgemisch wird auf 200 ml 2N-Salzsäure und Eis gegossen und zweimal mit Diäthyläther extrahiert. Die vereinigten Aetherextrakte werden mit Wasser neutral gewaschen und über Natriumsulfat getrocknet. Das Lösungsmittel wird im Vakuum abdestilliert und der Rückstand an Kieselgel chromatographisch gereinigt (Eluierungsmittel: Hexan/Diäthyläther, 9:1), wodurch reiner N-(4-Chlorphenylthio)-2-[4-(3,5-difluorphenoxy)-phenoxy]-äthylcarbaminsäure-äthylester erhalten wird, $n_D^{20}$ 1,5763

Beispiel H3: N-Phenylthio-2-[4-(3,5-difluorphenoxy)-phenoxy]-äthylcarbaminsäure-äthylester

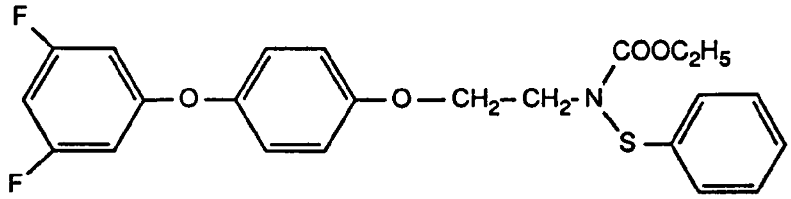

a) Zu einer Lösung von 66,5 g 2-Hydroxyäthylcarbaminsäure-äthylester, 43,5 g Pyridin und 1,9 g 4-Dimethylaminopyridin in 150 ml Methylenchlorid dosiert man unter Rühren in zirka 30 Minuten bei 0 bis +5°C 60 g Methansulfonsäurechlorid zu, rührt weitere 2 Stunden bei +5°C und anschliessend 16 Stunden bei Raumtemperatur. Das Reaktionsgemisch wird filtriert, der Rückstand mit wenig Essigsäureäthylester nachgewaschen und die vereinigten Filtrate mit verdünnter Salzsäure und Wasser gewaschen. Nach dem Trocknen über Magnesiumsulfat wird das Lösungsmittelgemisch im Vakuum vollständig abdestilliert, wodurch der 2-Methylsulfonyloxyäthylcarbaminsäure-äthylester erhalten wird, $n_D^{20}$ 1,4450.

b) 21,1 g des oben erhaltenen 2-Methansulfonyloxyäthylcarbaminsäure-äthylesters und 0,4 g 4-Dimethylaminopyridin werden in 48 g Pyridin gelöst und unter Rühren bei 0 bis +5°C innerhalb von 20 Minuten tropfenweise mit 15,8 g frisch destilliertem Phenylsulfenylchlorid in 15 ml Toluol versetzt. Für weitere 4 Stunden wird bei dieser Temperatur gerührt. Hierauf wird das Reaktionsgemisch auf 300 g Eis und 200 ml 2N-Salzsäure gegossen und mehrmals mit Aether extrahiert. Die vereinigten organischen Phasen werden mit gesättigter Natriumchlorid-Lösung neutral gewaschen und über Natriumsulfat getrocknet. Das Lösungsmittel wird im Vakuum abdestilliert. Durch chromatographische Reinigung an Kieselgel (Eluierungsmittel: Diäthyläther/Hexan, 1:1) wird der reine N-Phenylthio-2-methylsulfonyloxy-äthylcarbaminsäure-äthylester erhalten, $n_D^{20}$: 1,5390.

c) Zu einer Lösung von 6,5 g N-Phenylthio-2-methylsulfonyloxyäthyl-carbaminsäure-äthylester und 0,1 g Hydrochinon in 20 ml wasserfreiem Dimethylsulfoxid wird bei +25°C unter Rühren innerhalb von 5 Stunden die Lösung des Natriumsalzes von 4-(3,5-Difluorphenoxy)-phenol (hergestellt aus 4,3 g 4-(3,5-Difluorphenoxy)-phenol und 0,8 g 55%iger Natriumhydrid-Dispersion in Mineralöl in 40 ml trockenem Dimethylsulfoxid) gleichmässig zudosiert. Die Mischung wird für weitere 5 Stunden bei Raumtemperatur gerührt. Hierauf wird das Reaktionsgemisch auf Eiswasser gegossen, wiederholt mit einem Aether/Hexan-Gemisch (1:4) extrahiert, die vereinigten organischen Phasen mit Wasser gewaschen und über Natriumsulfat getrocknet. Nach dem Abdestillieren der Lösungsmittel im Vakuum wird das Rohprodukt durch Chromatographie gereinigt, wodurch der N-Phenylthio-2-[4-(3,5-difluorphenoxy)-phenoxy]-äthylcarbaminsäure-äthylester isoliert wird, $n_D^{20}$: 1,5700.

Beispiel H4: N-Phenylthio-2-[4-(3,5-fluorphenoxy)-phenoxy]-äthylcarbaminsäure-äthylester

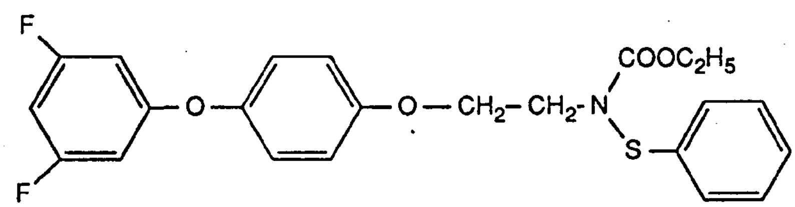

Zu einer Lösung von 5,8 g N-Phenylthio-2-chloräthylcarbaminsäure-äthylester, 0,1 g Kaliumjodid und 0,2 g Hydrochinon in 20 ml wasserfreiem Dimethylsulfoxid wird bei +25°C unter Rühren innerhalb von 5 Stunden die Lösung des Kaliumsalzes von 4-(4-Fluorphenoxy)-phenol (hergestellt aus 4,3 g 4-(3,5-Difluorphenoxy)-phenol und 2,04 g Kalium-tert.butylat in 35 ml trockenem Dimethylsulfoxid) gleichmässig zudosiert. Die Mischung wird für weitere 5 Stunden bei Raumtemperatur gerührt. Hierauf wird das Reaktionsgemisch auf Eiswasser gegossen, wiederholt mit einem Aether/Hexan-Gemisch (1:4) extrahiert. Die vereinigten organischen Phasen werden mit Wasser gewaschen und über Natriumsulfat getrocknet. Nach dem Abdestillieren der Lösungsmittel im Vakuum wird das Rohprodukt durch Chromatographie gereinigt, wodurch der N-Phenylthio-2-[4-(3,5-difluorphenoxy)phenoxy]-äthylcarbaminsäure-äthylester isoliert wird, $n_D^{20}$: 1,5700.

Beispiel H5: N-Phenylthio-2-[4-(3,5-difluorphenoxy)-phenoxy]-äthylcarbaminsäure-äthylester

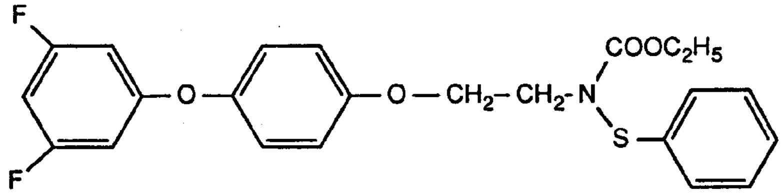

Zu einer Lösung von 6,4 g N-Phenylthio-2-methylsulfonyloxyäthylcarbaminsäureäthylester und 0,2 g Hydrochinon in 20 ml wasserfreiem Dimethylsulfoxid wird bei +25°C unter Rühren innerhalb von 5 Stunden die Lösung von 4,3 g 4-(3,5-Difluorphenoxy)phenol und 1,58 g Pyridin oder 2,58 g Aethyldiisopropylamin in 35 ml trockenem Dimethylsulfoxid gleichmässig zudosiert. Die Mischung wird für weitere 5 Stunden bei Raumtemperatur gerührt. Hierauf wird das Reaktionsgemisch auf Eiswasser gegossen, dreimal mit einem Aether/Hexan-Gemisch (1:4) extrahiert, die vereinigten organischen Phasen mit Wasser gewaschen und über Natriumsulfat getrocknet. Nach dem Abdestillieren der Lösungsmittel im Vakuum wird das Rohprodukt durch Chromatographie gereinigt, wodurch der N-Phenylthio-2-[4-(3,5-difluorphenoxy)-phenoxy]-äthylcarbaminsäure-äthylester isoliert wird, $n_D^{20}$ 1,5700.

Beispiel H6: N-Phenylsulfinyl-2-[4-(3,5-difluorphenoxy)-phenoxy]-äthylcarbaminsäure-äthylester

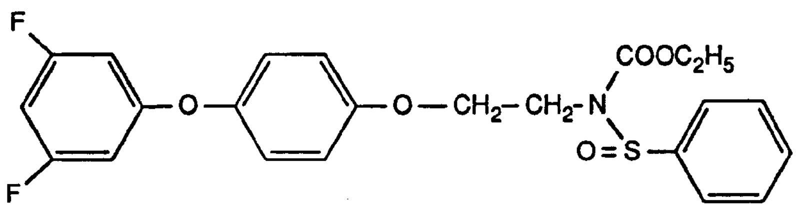

Zu einer Lösung von 11,5 g N-Phenylthio-2- [4-(3,5-difluorphenoxy)-phenoxy]-äthylcarbaminsäure-äthylester in 80 ml Dichlormethan lässt man unter Rühren bei 0° bis +5°C innerhalb von 20 Minuten eine Lösung von 8,1 g 55%iger 3-Chlorperbenzoesäure in 50 ml Dichlormethan zutropfen. Nach 2 Stunden Rühren bei +20° bis +22°C wird das Reaktionsgemisch zweimal mit 10%iger Natriumcarbonatlösung extrahiert und mit Wasser neutral gewaschen. Die Dichlormethanphase wird über Natriumsulfat getrocknet und das Lösungsmittel im Vakuum abdestilliert. Der Rückstand wird an Kieselgel chromatographisch gereinigt (Eluierungsmittel: n-Hexan/Diäthyläther, 3:1) wodurch der N-Phenylsulfinyl-2-[4-(3,5-difluorphenoxy)-phenoxy]-äthylcarbaminsäure-äthylester erhalten wird, farbloses viskoses Oel $n_D^{20}$: 1,5645

Beispiel H7: N-Phenylsulfonyl-2-[4-(3,5-difluorphenoxy)phenoxy]-äthylcarbaminsäure-äthylester

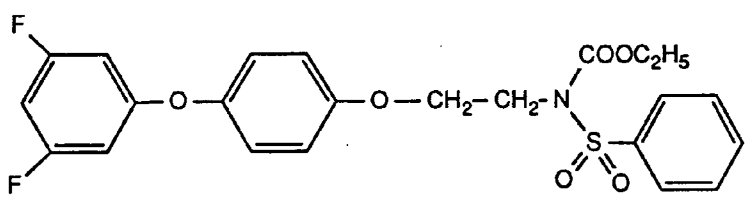

Zu einer Lösung von 4,8 g N-Phenylsulfinyl-2-[4-(3,5-difluorphenoxy)-phenoxy]-äthylcarbaminsäure-äthylester in 30 ml Dichlormethan lässt man unter Rühren bei zirka +5°C innerhalb von 10 Minuten die Lösung von 4,0 g 55%iger 3-Chlorperbenzoesäure in 30 ml Dichlormethan zutropfen und rührt für 16 Stunden bei Raumtemperatur. Das Reaktionsgemisch wird zweimal mit 10%iger Natriumcarbonat-Lösung und anschliessend mit Wasser neutralgewaschen. Die organische Phase wird über Magnesiumsulfat getrocknet und das Lösungsmittel im Vakuum abdestilliert. Das Rohprodukt wird durch Chromatographie an Kieselgel (Eluierungsmittel: n-Hexan/Diäthyläther, 3:1) weiter gereinigt, wodurch der N-Phenylsulfonyl-2-[4-(3,5-difluorphenoxy)-phenoxy]-äthylcarbaminsäure-äthylester als farblose harzige Masse isoliert wird, $n_D^{20}$: 1,5610.

Beispiel H8: N-Methylsulfonyl-2-[4-(3-chlorphenoxy)-phenoxy]-äthylcarbaminsäure-äthylester

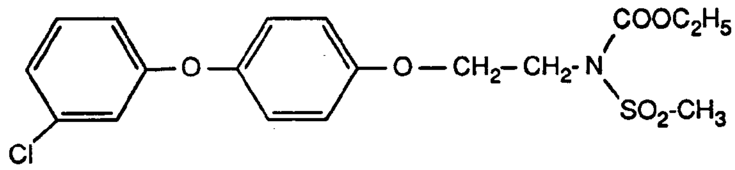

1,47 g einer 55%igen Natriumhydrid-Dispersion in Mineralöl werden wiederholt mit n-Hexan gewaschen und in 30 ml Tetrahydrofuran suspendiert. Zu dieser Suspension lässt man bei Raumtemperatur unter Rühren die Lösung von 11,3 g 2-[4-(3-Chlorphenoxy)phenoxy]-äthylcarbaminsäure-äthylester (Smp. 45-46°C; hergestellt aus 4- (3-Chlorphenoxy)-phenol und 2-Chloräthylcarbaminsäure-äthylester in Dimethylformamid analog Beispiel H1a) in 30 ml Tetrahydrofuran zutropfen und rührt bis zur vollständigen Umsetzung des Natriumhydrids ungefähr 5 Stunden bei Raumtemperatur. Hierauf lässt man bei 0°-5°C innerhalb von 10 Minuten eine Lösung von 4,2 g Methansulfosäurechlorid in 10 ml Tetrahydrofuran zutropfen und rührt weitere 15 Stunden bei Raumtemperatur. Nun wird das Reaktionsgemisch auf Eiswasser gegossen und wiederholt mit Aether extrahiert. Die vereinigten Aetherphasen wäscht man wiederholt mit 5%iger Natriumcarbonatlösung und anschliessend mit Wasser, trocknet die organische Phase über Natriumsulfat und destilliert das Lösungsmittel ab. Das Rohprodukt wird an Kieselgel chromatographisch weiter gereinigt (Eluierungsmittel: n-Hexa/Diäthyläther, 5:1), wodurch der N-Methylsulfonyl-2-[4-(3-chlorphenoxy)-phenoxy]-äthylcarbaminsäure-äthylester als farbloses, viskoses Oel erhalten wird, $n_D^{21}$: 1,5573.

Analog wird aus 2-[4-(3-Chlorphenoxy)-phenoxy]-äthylcarbaminsäure-äthylester und Benzolsulfosäurechlorid der N-Phenylsulfonyl-2-[4-(3-chlorphenoxy)-phenoxy]-äthylcarbaminsäure-äthylester als farbloses, viskoses Oel erhalten, $n_D^{20}$: 1,5790.

Beispiel H9: N-Phenylthio-2-[4-(3,5-difluorphenoxy)-phenoxy]-äthylcarbaminsäure-methylester

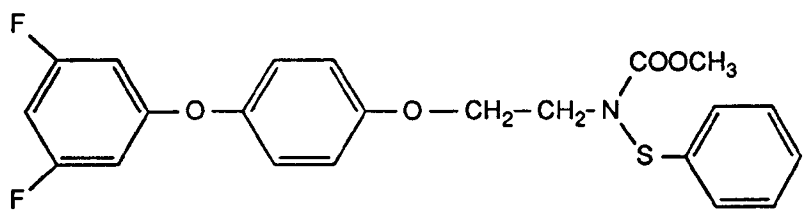

a) Zu einer Lösung von 17,1 g 4-(3,5-Difluorphenoxy)-phenol in 100 ml Dimethylformamid gibt man 21,3 g Kaliumcarbonat-Pulver, 1,5 g pulverisiertes Kaliumjodid und 16 g 2-Chloräthylcarbaminsäure-methylester zu und erhitzt das Reaktionsgemisch für 16 Stunden auf 95°C. Anschliessend wird das abgekühlte Reaktionsgemisch auf Eiswasser gegossen und wiederholt mit Diäthyläther extrahiert. Die vereinigten Aetherphasen werden mit Wasser gewaschen, über Natriumsulfat getrocknet und das Lösungsmittel abdestilliert. Das Rohprodukt wird an Kieselgel chromatographiert (Eluierungsmittel: n-Hexan/Diäthyläther, 5:1), wodurch der reine 2-[4-(3,5-Difluorphenoxy)-phenoxy]-äthylcarbaminsäure-methylester erhalten wird, $n_D^{21}$: 1,5394.

Analog erhält man aus den 2-Chloräthylcarbaminsäure-isopropyl- und -allylestern und 4-(3,5-Difluorphenoxy)-phenol:
2-[4-(3,5-Difluorphenoxy)-phenoxy]-äthylcarbaminsäure-isopropylester, $n_D^{21}$: 1,5243 und
2-[4-(3,5-Difluorphenoxy)-phenoxy]-äthylcarbaminsäure-allylester, Smp. 51-52°C.

b) Aus den unter a) erhaltenen Carbaminsäureestern erhält man durch Umsetzen mit Phenylsulfenylchlorid analog zur Arbeitsweise des Beispiels Hlb) die folgenden erfindungsgemässen Wirkstoffe:
N-Phenylthio-2-[4-(3,5-difluorphenoxy)-phenoxy]-äthylcarbaminsäure-methylester,
$n_D^{20}$: 1,5750;
N-Phenylthio-2-[4-(3,5-difluorphenoxy)-phenoxy]-äthylcarbaminsäure-isopropylester,

$n_D^{20}$: 1,5638 und
N-Phenylthio-2-[4-(3,5-Difluorphenoxy)-phenoxy]-äthylcarbaminsäure-allylester,
$n_D^{20}$: 1,6079.

Beispiel H10: N-Phenylthio-2-[4-(3-chlorphenoxy)-phenoxy]-1-methyl-äthylcarbaminsäure-äthylester

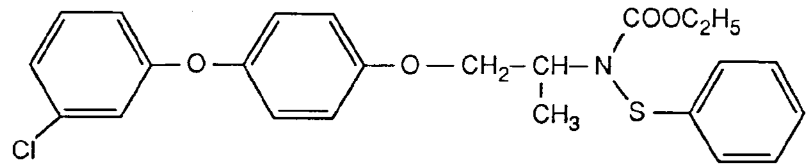

a) Zu einer Lösung von 39,8 g 4-(3-Chlorphenoxy)-phenol in 250 ml Aethylmethylketon gibt man 32,5 g wasserfreies Kaliumcarbonatpulver und 2 g fein pulverisiertes Kaliumjodid zu und erwärmt auf Rückflusstemperatur. Nun lässt man innerhalb von 45 Minuten 25 g frisch destilliertes Chloraceton zutropfen und rührt weitere 2 Stunden bei Rückflusstemperatur. Nach dem Abkühlen wird das Reaktionsgemisch filtriert, das Lösungsmittel im Vakuum abdestilliert und der Rückstand im Hochvakuum bei 40°C vollständig vom Lösungsmittel befreit. Das so erhaltene analysenreine, ölartige 4-(3-Chlorphenoxy)-phenoxy-propanon, $n_D^{20}$: 1,5788, wird direkt weiter umgesetzt.
b) 51 g 4-(3-Chlorphenoxy)-phenoxy-propanon werden in einem Rührautoklaven in 510 ml Methanol gelöst. 31 g flüssiges Ammoniak wird aufgepresst und die Mischung in Gegenwart von 10 g Raney-Nickel und 50 bar Wasserstoffdruck bei 40-45°C für 3 Stunden der reduktiven Aminierung unterworfen. Nach dem Entspannen des Autoklaven wird das Raney-Nickel vom Reaktionsgemisch abfiltriert. Der Katalysator wird mit Methanol nachgewaschen und schliesslich das Methanol aus dem Reaktionsgemisch abdestilliert. Der Rückstand wird an Kieselgel chromatographiert (Eluierungsmittel: Diäthyläther(Methanol, 5:1), wodurch das reine 1-[4-(3-Chlorphenoxy)-phenoxy]-2-aminopropan erhalten wird, $n_D^{20}$ 1,5743.
c) Zu einer Lösung von 31,5 g 1-[4-(3-Chlorphenoxy)-phenoxy]-2-aminopropan, 20 g Diisopropyläthylamin und 1 g 4-Dimethylaminopyridin in 120 ml Toluol lässt man unter Rühren und leichter Aussenkühlung bei 20°C innerhalb von 30 Minuten 13,5 g Chlorameisensäureäthylester zutropfen und rührt weitere 15 Stunden bei 20°C. Hierauf wird das Reaktionsgemisch in 200 ml 2N-Salzsäure und Eiswasser gegossen und die Toluolphase abgetrennt. Die wässrige Phase wird nochmals mit Aether extrahiert und die vereinigten organischen Phasen mit Wasser und Natriumchloridlösung neutral gewaschen, über Natriumsulfat getrocknet und die Lösungsmittel im Vakuum abdestilliert. Das Rohprodukt wird an Kieselgel (Eluierungsmittel: n-Hexan/Diäthyläther, 5:1) chromatographiert, wodurch der reine 2-[4-(3-Chlorphenoxy)-phenoxy]-1-methyl-äthylcarbaminsäure-äthylester als blassgelbes Oel erhalten wird, $n_D^{21}$: 1,5530.
d) Zu einer Lösung von 9 g 2-[4-(3-Chlorphenoxy)-phenoxy]-1-methyl-äthylcarbaminsäure-äthylester in 30 ml Pyridin lässt man unter Rühren bei 0-5°C innerhalb von 20 Minuten die Lösung von 4 g Phenylsulfenylchlorid in 10 ml Toluol zutropfen und rührt weitere 15 Stunden bei Raumtemperatur. Anschliessend wird das Pyridin und Toluol im Vakuum bei einer Temperatur von unter 45°C grösstenteils abdestilliert und der Rückstand auf 300 ml Eiswasser gegossen und wiederholt mit Aether extrahiert. Die vereinigten Aetherphasen wäscht man erst mit kalter, verdünnter Salzsäure anschliessend mit Wasser, trocknet über Natriumsulfat und destilliert das Lösungsmittel vollständig ab. Der Rückstand wird durch Chromatographie an Kieselgel weiter gereinigt (Eluierungsmittel: n-Hexan(Diäthyläther, 19:1), wodurch der N-Phenylthio-2-[4-(3-chlorphenoxy)-phenoxy]-1-methyl-äthylcarbaminsänre-äthylester als farbloses Oel erhalten wird, $n_D^{20}$: 1,5810.
In analoger Weise kann man die folgenden Wirkstoffe der Formel I enthalten:

Tabelle 1:

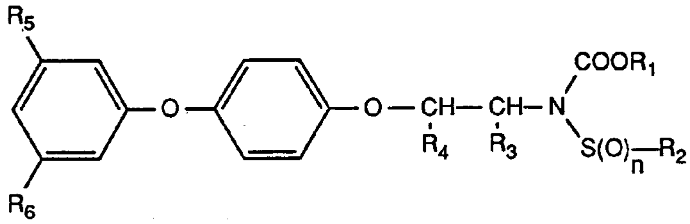

| Verb. Nr. | $R_1$ | $R_2$ | n | $R_3$ | $R_4$ | $R_5$ | $R_6$ | Physikalische Daten |
|---|---|---|---|---|---|---|---|---|
| 1.01 | $C_2H_5$ | $C_6H_5$ | 0 | H | H | F | F | $n_D^{20}$: 1,5700 |
| 1.02 | $C_2H_5$ | $C_6H_5$ | 1 | H | H | F | F | $n_D^{20}$: 1,5645 |
| 1.03 | $C_2H_5$ | $C_6H_5$ | 2 | H | H | F | F | $n_D^{20}$: 1,5610 |
| 1.04 | $C_2H_5$ | 4-Cl-$C_6H_4$- | 0 | H | H | F | F | $n_D^{20}$: 1,5763 |
| 1.05 | $C_2H_5$ | 4-$CH_3$-$C_6H_4$- | 0 | H | H | F | F | $n_D^{20}$: 1,5705 |
| 1.06 | $C_2H_5$ | 3-$CH_3$-$C_6H_4$- | 0 | H | H | F | F | |
| 1.07 | $C_2H_5$ | 2-$CH_3$-$C_6H_4$- | 0 | H | H | F | F | |
| 1.08 | $C_2H_5$ | 3-Cl-$C_6H_4$- | 0 | H | H | F | F | |
| 1.09 | $C_2H_5$ | 3-Cl-4-Cl-$C_6H_4$- | 0 | H | H | F | F | |
| 1.10 | $C_2H_5$ | 4-$NO_2$-$C_6H_4$- | 0 | H | H | F | F | |
| 1.11 | $C_2H_5$ | $C_6H_5$-$CH_2$- | 0 | H | H | F | F | |
| 1.12 | $CH_3$ | $C_6H_5$ | 0 | H | H | F | F | $n_D^{20}$: 1,5750 |
| 1.13 | $C_4H_9$-n | $C_6H_5$ | 0 | H | H | F | F | |
| 1.14 | $C_2H_5$ | $C_6H_5$ | 0 | $CH_3$ | H | Cl | H | $n_D^{20}$: 1,5810 |
| 1.15 | $C_2H_5$ | $C_6H_5$ | 1 | $CH_3$ | H | F | F | |
| 1.16 | $C_2H_5$ | $C_6H_5$ | 1 | H | H | F | F | |
| 1.17 | $C_2H_5$ | 4-$CH_3$-$C_6H_4$- | 1 | H | H | F | F | |
| 1.18 | $C_2H_5$ | $C_6H_5$ | 0 | H | H | F | H | |
| 1.19 | $C_2H_5$ | 4-Cl-$C_6H_4$- | 0 | H | H | F | H | |
| 1.20 | $C_2H_5$ | $C_6H_5$-$CH_2$- | 0 | H | H | F | H | |
| 1.21 | $C_2H_5$ | $C_6H_5$ | 1 | H | H | F | H | |

Tabelle 1 (Fortsetzung)

| Verb. Nr. | $R_1$ | $R_2$ | n | $R_3$ | $R_4$ | $R_5$ | $R_6$ | Physikalische Daten |
|---|---|---|---|---|---|---|---|---|
| 1.22 | $C_2H_5$ | $C_6H_5$ | 0 | H | H | Cl | H | $n_D^{21}$: 1,5851 |
| 1.23 | $C_2H_5$ | $C_6H_5$ | 1 | H | H | Cl | H | |
| 1.24 | $C_2H_5$ | 4-Cl-$C_6H_4$- | 0 | H | H | Cl | H | $n_D^{20}$: 1,5879 |
| 1.25 | $C_2H_5$ | 4-$CH_3$-$C_6H_4$- | 0 | H | H | Cl | H | $n_D^{20}$: 1,5934 |
| 1.26 | $C_2H_5$ | 4-$CH_3$-$C_6H_4$- | 0 | H | H | Cl | Cl | |
| 1.27 | $C_2H_5$ | 4-Cl-$C_6H_4$- | 0 | H | H | Cl | Cl | |
| 1.28 | $C_2H_5$ | $C_6H_5$ | 0 | H | H | Cl | Cl | |
| 1.29 | $C_2H_5$ | $C_6H_5$ | 1 | H | H | Cl | Cl | |
| 1.30 | $C_2H_5$ | $C_6H_5$ | 0 | $(CH_2)_4$ | | Cl | Cl | |
| 1.31 | $C_2H_5$ | 2-$CH_3$-4-$CH_3$-$C_6H_3$- | 0 | H | H | F | F | $n_D^{20}$: 1,5713 |
| 1.32 | $C_2H_5$ | $CH_3$ | 2 | H | H | Cl | H | $n_D^{21}$: 1,5573 |
| 1.33 | $C_3H_7$-i | $C_6H_5$ | 0 | H | H | F | F | $n_D^{20}$: 1,5638 |
| 1.34 | $C_2H_5$ | 4-Cl-$C_6H_4$- | 2 | H | H | F | F | $n_D^{22}$: 1,5641 |
| 1.35 | $C_2H_5$ | $C_6H_5$ | 2 | H | H | Cl | H | $n_D^{20}$: 1,5790 |
| 1.36 | $-CH_2CH=CH_2$ | $C_6H_5$ | 0 | H | H | F | F | $n_D^{20}$: 1,6079 |
| 1.37 | $C_2H_5$ | $C_6H_5$ | 0 | $CH_3$ | H | F | F | |
| 1.38 | $-CH_2-CH=CH_2$ | $C_6H_5$ | 0 | H | H | Cl | H | |

Formulierungsbeispiele für flüssige Wirkstoffe der Formel I (% = Gewichtsprozent)

| F1. Emulsions-Konzentrate | a) | b) | c) |
|---|---|---|---|
| Wirkstoff Nr. 1.01 | 25 % | 40 % | 50 % |
| Ca-Dodecylbenzolsulfonat | 5 % | 8 % | 6 % |
| Ricinusölpolyäthylenglykoläther (36 Mol AeO) | 5 % | - | - |
| Tributylphenolpolyäthylenglykoläther (30 Mol AeO) | - | 12 % | 4 % |
| Cyclohexanon | - | 15 % | 20 % |
| Xylolgemisch | 65 % | 25 % | 20 % |

Aus solchen Konzentraten können durch Verdünnen mit Wasser Emulsionen jeder gewünschten Konzentration hergestellt werden.

| F2. Lösungen | a) | b) | c) | d) |
|---|---|---|---|---|
| Wirkstoff Nr. 1.02 | 80 % | 10 % | 5 % | 95 % |
| Aethylenglykolmonomethyläther | 20 % | - | - | - |
| Polyäthylenglykol MG 400 | - | 70 % | - | - |
| N-Methyl-2-pyrrolidon | - | 20 % | - | - |
| Epoxydiertes Kokosnussöl | - | - | 1 % | 5 % |
| Benzin (Siedegrenzen 160-190°C) | - | - | 94 % | - |

Die Lösungen sind zur Anwendung in Form kleinster Tropfen geeignet.

| F3. Granulate | a) | b) |
|---|---|---|
| Wirkstoff Nr. 1.02 | 5 % | 10 % |
| Kaolin | 94 % | - |
| Hochdisperse Kieselsäure | 1 % | - |
| Attapulgit | - | 90 % |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff Nr. 1.01 | 2 % | 5 % |
| Hochdisperse Kieselsäure | 1 % | 5 % |
| Talkum | 97 % | - |
| Kaolin | - | 90 % |

Durch inniges Vermischen der Trägerstoffe mit dem Wirkstoff erhält man gebrauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff 1.03 | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

| F6. Emulsions-Konzentrat | |
|---|---|
| Wirkstoff Nr. 1.03 | 10 % |
| Octylphenolpolyäthylenglykoläther (4-5 Mol AeO) | 3 % |
| Ca-Dodecylbenzolsulfonat | 3 % |
| Ricinusölpolyglykoläther (36 Mol AeO) | 4 % |
| Cyclohexanon | 30 % |
| Xylolgemisch | 50 % |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff Nr. 1.03 | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

| F8. Extruder-Granulat | |
|---|---|
| Wirkstoff Nr. 1.03 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

F9. Umhüllungs-Granulat

| | |
|---|---|
| Wirkstoff Nr. 1.03 | 3 % |
| Polyäthylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff Nr. 1.03 | 40 % |
| Aethylenglykol | 10 % |
| Nonylphenolpolyäthylenglykoläther (15 Mol AeO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

In den nachfolgenden biologischen Beispielen bedeutet eine gute Wirkung, dass der erwünschte Effekt zu mindestens 50 bis 60 % eintritt.

Beispiel B1: Wirkung gegen Boophilus microplus

Vollgesogene adulte Zeckenweibchen werden auf eine PVC Platte aufgeklebt und mit einem Wattebausch überdeckt. Zur Behandlung werden die Testtiere mit 10 ml einer wässrigen Testlösung, die 125 ppm des zu prüfenden Wirkstoffes enthält übergossen. Anschliessend wird der Wattebausch entfernt und die Zecken werden für 4 Wochen zur Eiablage inkubiert. Die Wirkung gegen Boophilus microplus zeigt sich entweder beim Weibchen als Mortalität oder Sterilität, oder bei den Eiern als ovizide Wirkung.

Verbindungen der Tabelle 1 zeigen in diesem Test gute Wirkung gegen Boophilus microplus. Insbesondere die Verbindungen 1.01, 1.03 und 1.33 zeigen eine Wirkung über 80%.

Beispiel B2: Wirkung gegen Aedes aegypti

50-100 Larven von Aedes aegypti werden in 200 ml einer wässrigen Testlösung, die 400 ppm des zu prüfenden Wirkstoffes und sehr wenig Futter enthält, gegeben. Anschliessend wird das Testgefäss mit einem Deckel verschlossen und inkubiert. Die Auswertung erfolgt 14 Tage nach dem Ansatz auf %-Schlupf der Adulten im Vergleich zu unbehandelten Kontrollansätzen.

Verbindungen gemäss Tabelle 1 zeigen in diesem Test gute Wirkung gegen Aedes aegypti. Insbesondere die Verbindungen 1.04, 1.22, 1.24 und 1.25 zeigen eine Wirkung über 80 %.

Beispiel B3: Ovizide Wirkung auf Cydia pomonella

Auf Filterpapier abgelegte Eier von Cydia pomonella werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf aus den Eiern im Vergleich zu unbehandelten Kontrollansätzen ausgewertet (% Schlupfreduktion).

Verbindungen gemäss Tabelle 1 zeigen in diesem Test gute Wirkung gegen Cydia pomonella. Insbesondere die Verbindungen 1.01, 1.04 und 1.05 zeigen eine Wirkung über 80 %.

Beispiel B4: Ovizide Wirkung auf Adoxophyes reticulana

Auf Filterpapier abgelegte Eier von Adoxophyes reticulana werden für kurze Zeit in eine acetonisch-wässrige Testlösung, die 400 ppm des zu prüfenden Wirkstoffes enthält, eingetaucht. Nach dem Antrocknen der Testlösung werden die Eier in Petrischalen inkubiert. Nach 6 Tagen wird der prozentuale Schlupf der Eier im Vergleich zu unbehandelten Kontrollansätzen ausgewertet (% Schlupfreduktion).

Verbindungen gemäss Tabelle 1 zeigen in diesem Test gute Wirkung gegen Adoxophyes reticulana. Insbesondere die Verbindungen 1.01, 1.05 und 1.12 zeigen eine Wirkung über 80 %.

Beispiel B5: Ovizide Wirkung gegen Dermanyssus gallinae

In einem nach oben offenen Glasbehälter werden 2 bis 3 ml einer 10 ppm Wirkstoff enthaltenden Lösung und ca. 200 Milben in unterschiedlichen Entwicklungsstadien gegeben. Anschliessend wird der Behälter mit einem Wattebausch verschlossen, 10 Minuten lang bis zur vollständigen Benetzung der Milben geschüttelt und dann kurzfristig umgekehrt, damit die restliche Testlösung von der Watte aufgenommen werden kann. Nach 3 Tagen wird die Mortalität der Milben ermittelt.

Die Verbindungen der Tabelle 1 zeigen eine gute Wirkung gegen Dermanyssus gallinae. Insbesondere die Verbindungen 1.01, 1.05 und 1.22 zeigen eine Wirkung über 80 %.

Beispiel B6: Wirkung gegen Aonidiella aurantii

Kartoffelknollen werden mit Wanderlarven ("Crawlern") von Aonidiella aurantii (rote Citrus-Schildlaus) besiedelt. Nach etwa 2 Wochen werden die Kartoffeln in eine wässrige Emulsions-Spritzbrühe getaucht, die den zu prüfenden Wirkstoff in einer Konzentration von 400 ppm enthält. Nach dem Abtrocknen der so behandelten Kartoffelknollen werden diese in einem Plastikbehälter inkubiert. Zur Auswertung wird 10-12 Wochen später die Ueberlebensrate der Wanderlarven der ersten Folgegeneration der behandelten Schildlaus-Population mit derjenigen von unbehandelten Kontrollansätze verglichen.

Verbindungen gemäss Tabelle 1 zeigen in diesem Test gute Wirkung gegen Aonidiella aurantii. Insbesondere die Verbindungen 1.01, 1.04 und 1.31 zeigen eine Wirkung über 80 %.

Beispiel B7: Wirkung gegen Nilaparvata lugens

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit adulten Zikaden zwecks Eiablage besiedelt. Nach der Eiablage werden die Adulten entfernt und die Pflanzen inkubiert. 14 Tage später erfolgt die Auswertung. Aus dem Vergleich der Anzahl geschlüpfter Nymphen auf den behandelten zu denjenigen auf unbehandelten Pflanzen wird die prozentuale Reduktion der Nachkommenschaft (% Wirkung) bestimmt.

Die Verbindungen der Tabelle 1 zeigen eine gute Wirkung gegen Nilaparvata lugens in diesem Test. Insbesondere die Verbindungen 1.01, 1.02, 1.04, 1.22, 1.24, 1.25 und 1.31 zeigen eine Wirkung über 80 %.

Beispiel B8: Wirkung gegen Nephotettix cincticeps

Reispflanzen werden mit einer wässrigen Emulsions-Spritzbrühe, die 400 ppm des Wirkstoffes enthält, behandelt. Nach dem Antrocknen des Spritzbelages werden die Reispflanzen mit adulten Zikaden zwecks Eiablage besiedelt. Nach der Eiablage werden die Adulten entfernt und die Pflanzen inkubiert. 14 Tage später erfolgt die Auswertung. Aus dem Vergleich der Anzahl geschlüpfter Nymphen auf den behandelten zu denjenigen auf den unbehandelten Pflanzen wird die prozentuale Reduktion der Nachkommenschaft (% Wirkung) bestimmt.

Die Verbindungen der Tabelle 1 zeigen eine gute Wirkung gegen Nephotettix cincticeps in diesem Test. Insbesondere die Verbindung 1.01 zeigt eine Wirkung über 80 %.

Beispiel B9: Wirkung geben Bemisia tabaci

Buschbohnen werden in Gazekäfige gestellt und mit Adulten von Bemisia tabaci (Weisse Fliege) besiedelt. Nach erfolgter Eiablage werden alle Adulten entfernt und 10 Tage später die Pflanzen mit den darauf befindenden Nymphen mit einer wässrigen Emulsions-Spritzbrühe der zu prüfenden Wirkstoffe (Konzentration 400 ppm) behandelt. Die Auswertung erfolgt 14 Tage nach der Wirkstoff-Applikation auf %-Schlupf im Vergleich zu unbehandelten Kontrollansätzen.

Verbindungen gemäss Tabelle 1 zeigen in diesem Test gute Wirkung gegen Bemisia tabaci. Insbesondere die Verbindungen 1.01, 1.04, 1.22, 1.24 und 1.25 zeigen eine Wirkung über 80 %.

## Patentansprüche

### Patentansprüche für folgende Vertragsstaaten : AT, BE, CH, DE, DK, FR, GB, IT, LI, NL

**1.** 2-Phenoxyphenoxy-äthylcarbaminsäureester der Formel

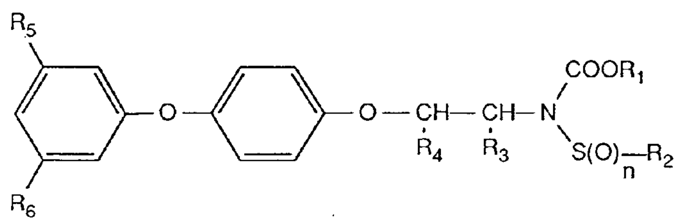

(I),

worin
$R_1$ $C_1$-$C_4$-Alkyl oder $C_3$-$C_4$-Alkenyl,
$R_2$ $C_1$-$C_4$-Alkyl oder einen Rest der Formel

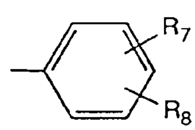

,

$R_3$ Wasserstoff oder Methyl, $R_4$ Wasserstoff,
$R_5$ und $R_6$ entweder beide Fluor oder $R_5$ Chlor und $R_6$ Wasserstoff,
$R_7$ und $R_8$ unabhängig voneinander Wasserstoff, Halogen oder $C_1$-$C_4$-Alkyl,
und
n null, eins oder zwei
bedeuten.

**2.** Eine Verbindung gemäss Anspruch 1, ausgewählt aus der Gruppe, bestehend aus

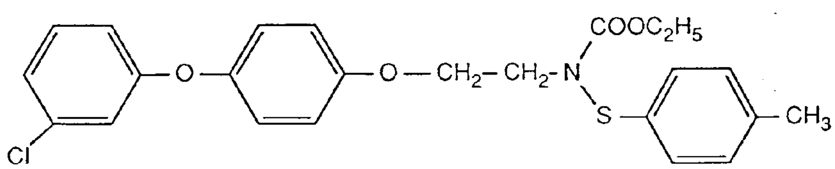

,

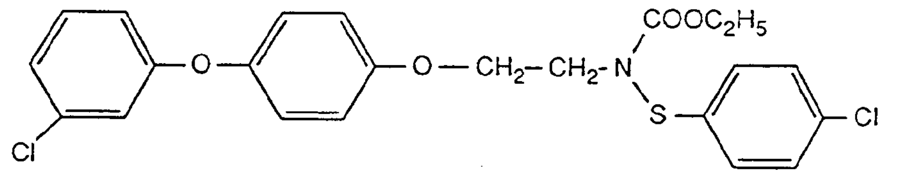

,

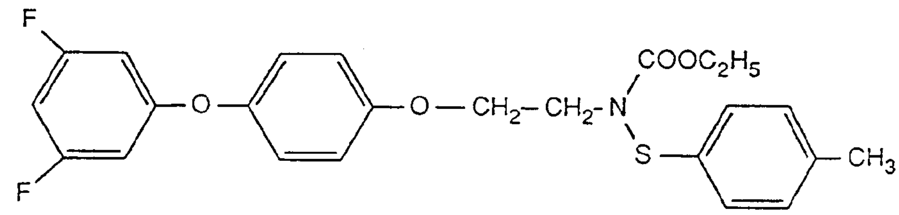

,

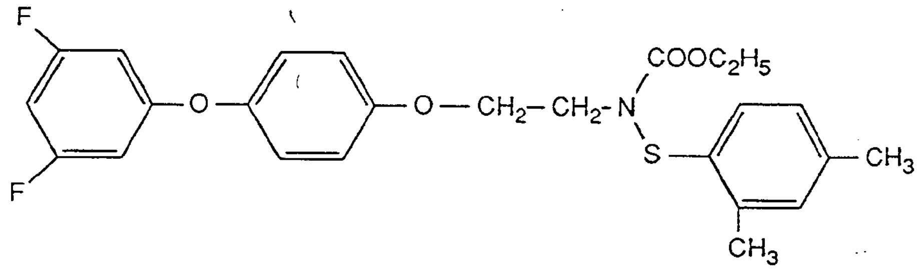

,

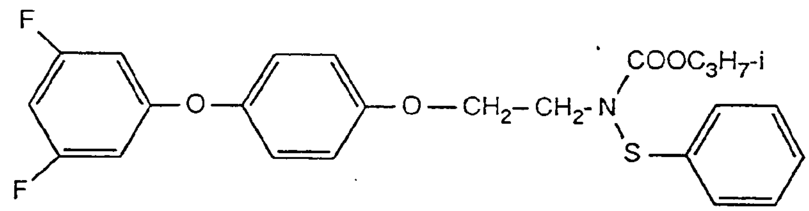

,

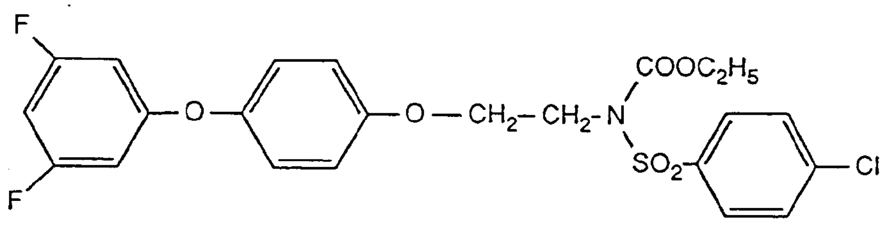

,

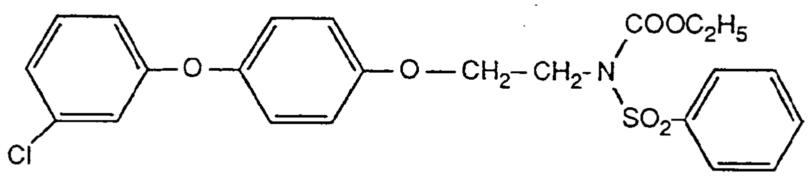

,

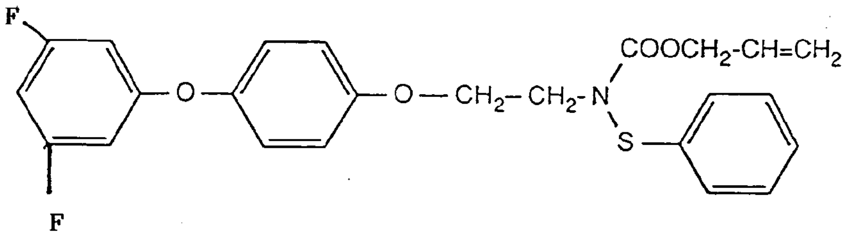

,

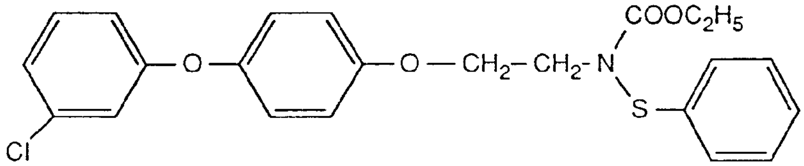

,

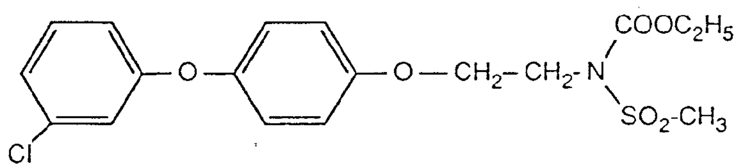

,

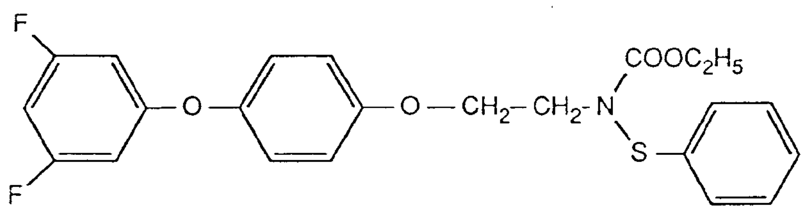

,

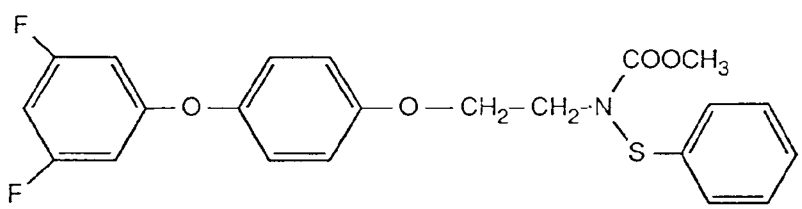

,

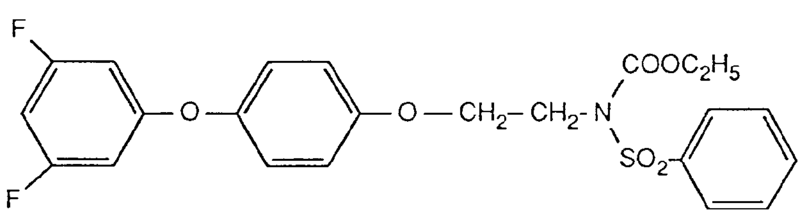

,

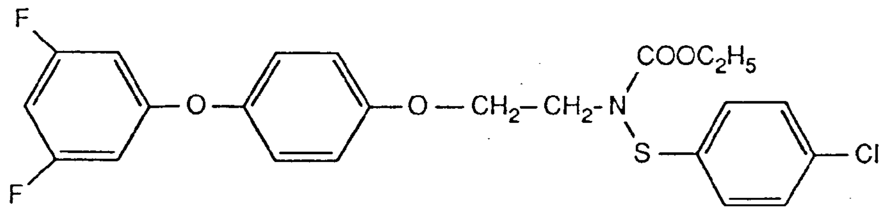

und

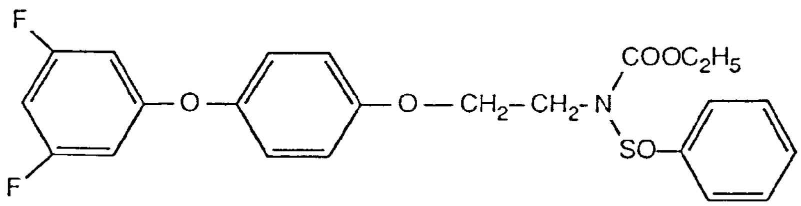

3. Die Verbindung der formel

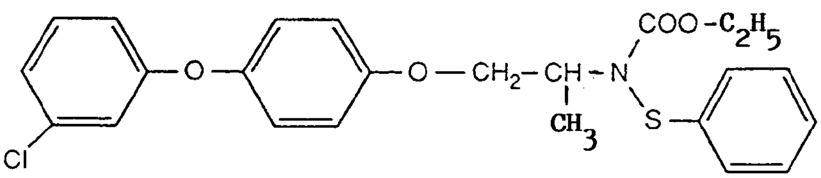

gemäss Anspruch 1.

4. Verfahren zur Herstellung einer Verbindung der Formel I gemäss Anspruch 1, dadurch gekennzeichnet, dass man entweder
   a) ein Phenoxyphenol der Formel

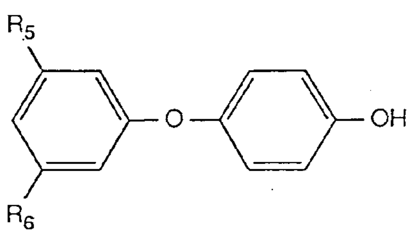

(II),

worin $R_5$ und $R_6$ die unter Formel I gegebenen Bedeutungen haben, in Gegenwart einer Base mit einem Aethylcarbaminsäure-Derivat der Formel

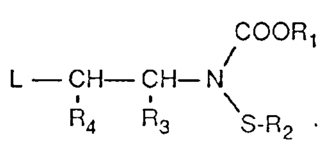

(III),

worin $R_1$, $R_2$, $R_3$ und $R_4$ die unter Formel I gegebenen Bedeutungen haben und L für eine Abgangsgruppe steht, umsetzt und das erhaltene Produkt der Formel

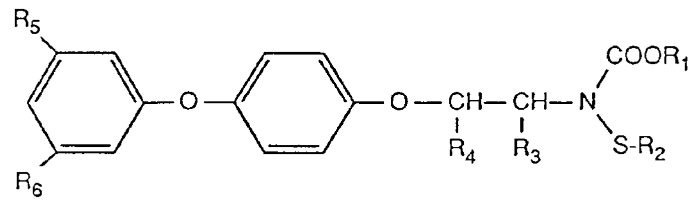

(Ia),

worin $R_1$, $R_2$, $R_3$, $R_4$, $R_5$ und $R_6$ die unter Formel I gegebenen Bedeutungen haben, gewünschtenfalls zu einer Verbindung der Formel I, worin n eins oder zwei bedeutet, oxidiert, oder,
b) ein Aethylcarbaminsäure-Derivat der Formel

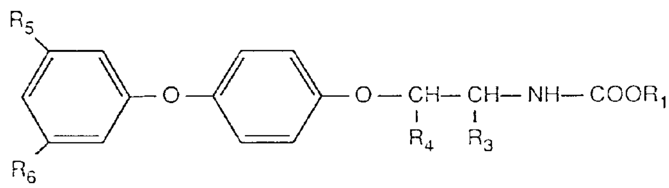

(IV),

worin $R_1$, $R_3$, $R_4$, $R_5$ und $R_5$ die unter Formel I gegebenen Bedeutungen haben, in Gegenwart einer Base mit einem Sulfensäurehalogenid der Formel

$$Hal\text{-}S\text{-}R_2 \quad (V),$$

worin $R_2$ die unter Formel I gegebene Bedeutung hat und Hal für Halogen steht, umsetzt und das erhaltene Produkt der Formel Ia gewünschtenfalls zu einer Verbindung der Formel I, worin n eins oder zwei bedeutet, oxidiert; oder
c) ein Aethylcarbaminsäure-Derivat der Formel IV in Gegenwart einer Base mit einem Sulfinsäurehalogenid der Formel

$$Hal\text{-}SO\text{-}R_2 \quad (VI),$$

worin $R_2$ die unter Formel I gegebene Bedeutung hat und Hal für Halogen steht, umsetzt und das erhaltene Produkt der Formel

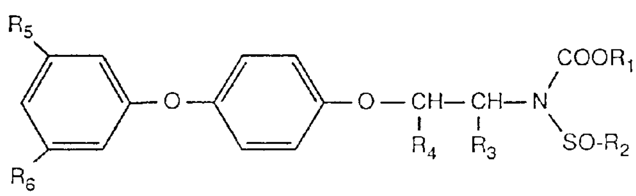

(Ib),

worin $R_1$, $R_2$, $R_3$, $R_4$, $R_5$ und $R_6$ die unter Formel I gegebenen Bedeutungen haben, gewünschtenfalls zu einer Verbindung der Formel I, worin n zwei bedeutet, oxidiert; oder
d) ein Aethylcarbaminsäure-Derivat der Formel IV in Gegenwart einer Base mit einem Sulfonsäurehalogenid der Formel

$$Hal\text{-}SO_2\text{-}R_2 \quad (VII),$$

worin $R_2$ die unter Formel I gegebene Bedeutung hat und Hal für Halogen steht, umsetzt zu einer Verbindung der Formel

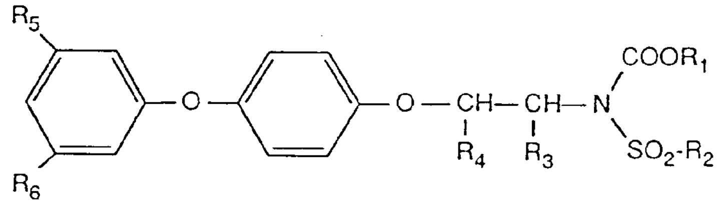

(Ic),

worin $R_1$, $R_2$, $R_3$, $R_4$, $R_5$ und $R_6$ die unter Formel I gegebenen Bedeutungen haben; oder
e) ein Aethylaminderivat der Formel

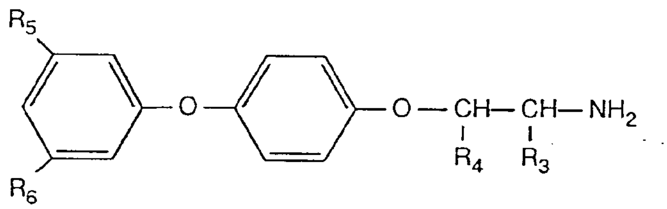

(VIII),

worin $R_3$, $R_4$, $R_5$ und $R_6$ die unter Formel I gegebenen Bedeutungen haben, in Gegenwart einer Base mit einem Halogenameisensäurederivat der Formel

Hal-$COOR_1$ (IX),

worin $R_1$ die unter Formel I gegebene Bedeutung hat und Hal für Halogen steht, acyliert und das erhaltene Zwischenprodukt der Formel IV in Gegenwart einer Base mit einem Sulfensäurehalogenid der Formel V sulfenyliert und das erhaltene Produkt der Formel Ia gewünschtenfalls zu einer Verbindung der Formel I, worin n eins oder zwei bedeutet, oxidiert; oder
f) ein Aethylaminderivat der Formel VIII in Gegenwart einer Base mit einem Sulfensäurehalogenid der Formel V sulfeniert und das erhaltene Zwischenprodukt der Formel

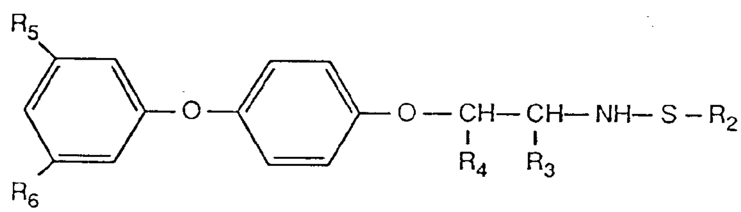

(X),

worin $R_2$, $R_3$, $R_4$, $R_5$ und $R_6$ die unter Formel I gegebenen Bedeutungen haben, in Gegenwart einer Base mit einem Halogenameisensäurederivat der Formel IX acyliert und das erhaltene Produkt der Formel Ia gewünschtenfalls zu einer Verbindung der Formel I, worin n eins oder zwei bedeutet, oxidiert; oder
g) zur Herstellung einer Verbindung der Formel Ic ein Alkalisalz einer Verbindung der Formel

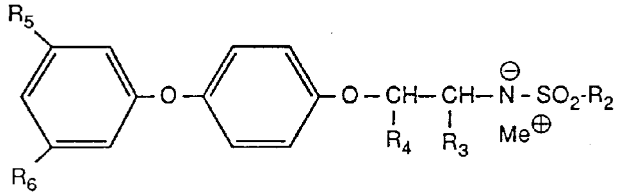

(Xa)

mit einer Verbindung der Formel IX umsetzt, wobei $R_1$ bis $R_6$ die unter Formel I angegebenen Bedeu-

tungen haben und $Me^{\oplus}$ ein Alkalimetallkation bedeutet.

5. Schädlingsbekämpfungsmittel, welches als aktive Komponente mindestens eine Verbindung der Formel I gemäss Anspruch 1 enthält.

6. Mittel gemäss Anspruch 5 dadurch gekennzeichnet, dass es zusätzlich mindestens einen Träger enthält.

7. Verwendung einer Verbindung der Formel I gemäss Anspruch 1 zur Bekämpfung von Schädlingen an Pflanzen und Tieren, im Vorratsschutz und in der Hygiene.

8. Verwendung gemäss Anspruch 7 zur Bekämpfung von Arthropoden, insbesondere von Insekten und Arachniden.

9. Verwendung gemäss Anspruch 8 zur Bekämpfung von Larven und Eiern phytophager Schadinsekten und -milben.

10. Verwendung gemäss Anspruch 8 zur Abtötung von Eiern pflanzenschädigender Insekten und Ektoparasiten.

11. Verwendung gemäss Anspruch 8 zur Hemmung der Entwicklung von Larven pflanzenschädigender Insekten und Ektoparasiten.

**Patentansprüche für folgenden Vertragsstaat : ES**

1. Schädlingsbekämpfungsmittel, welches als aktive Komponente mindestens einen 2-Phenoxyphenoxyäthylcarbaminsäureester der Formel

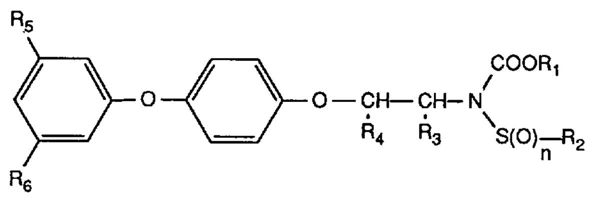

(I),

worin
$R_1$ $C_1$-$C_4$-Alkyl oder $C_3$-$C_4$-Alkenyl,
$R_2$ $C_1$-$C_4$-Alkyl oder einen Rest der Formel

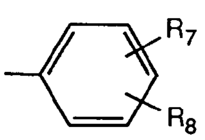

,

$R_3$ Wasserstoff oder Methyl, $R_4$ Wasserstoff,
$R_5$ und $R_6$ entweder beide Fluor oder $R_5$ Chlor und $R_6$ Wasserstoff,
$R_7$ und $R_8$ unabhängig voneinander Wasserstoff, Halogen oder $C_1$-$C_4$-Alkyl,
und
n null, eins oder zwei bedeuten, enthält.

2. Mittel gemäss Anspruch 1, dadurch gekennzeichnet, dass es zusätzlich mindestens einen Träger enthält.

3. Mittel gemäss Anspruch 1 enthaltend einen Wirkstoff ausgewählt aus der Gruppe

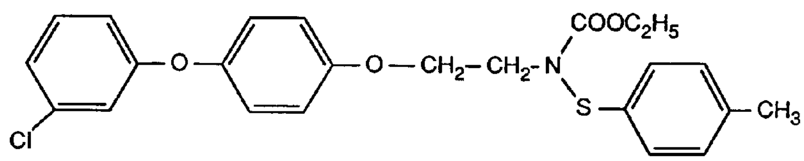

,

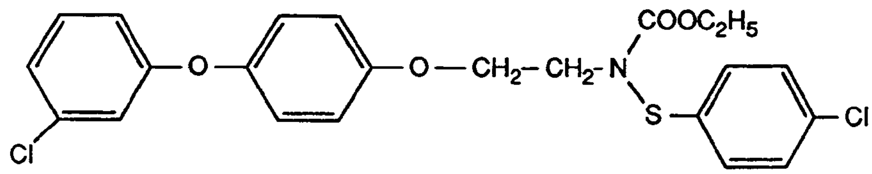

,

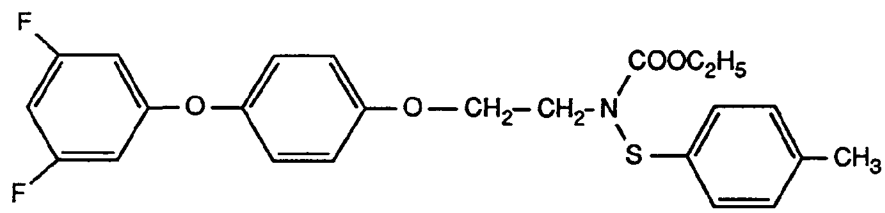

,

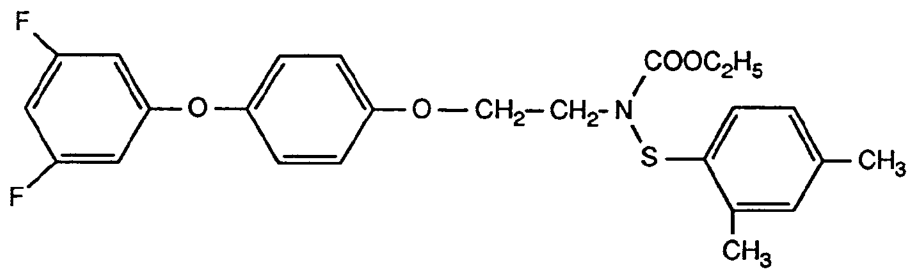

,

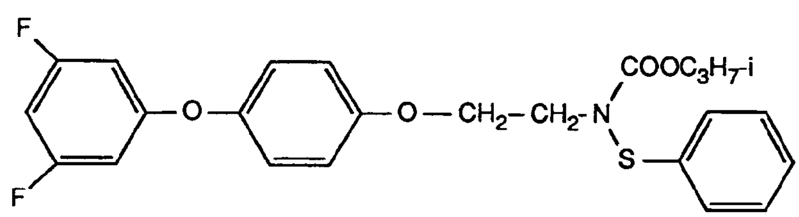

,

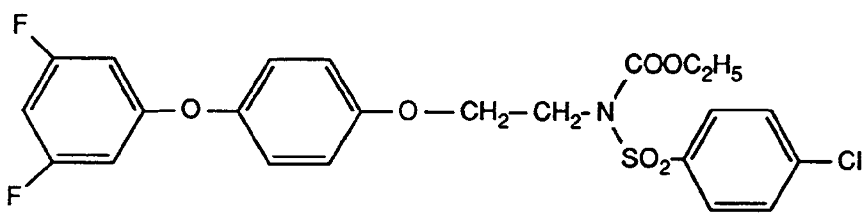

,

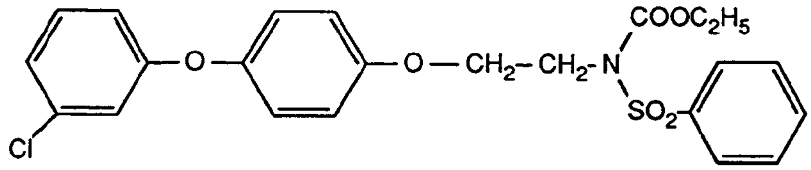

,

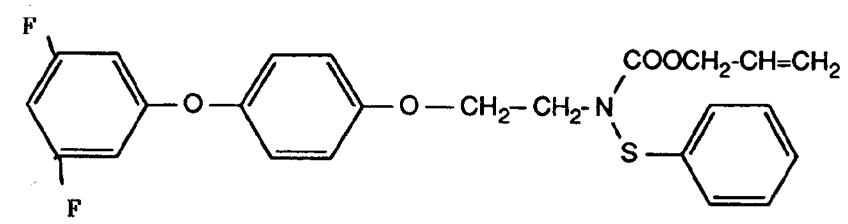

,

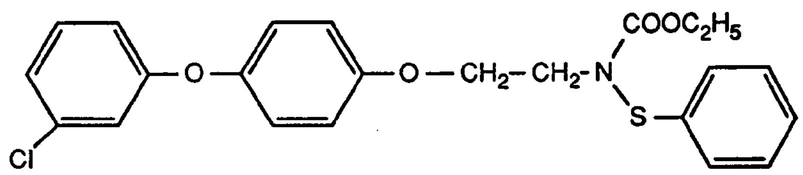

,

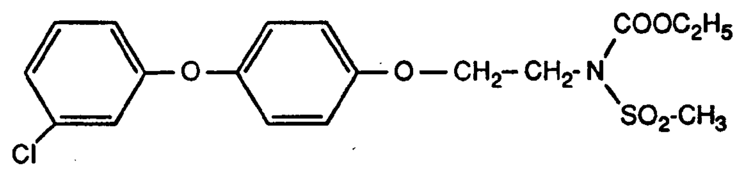

,

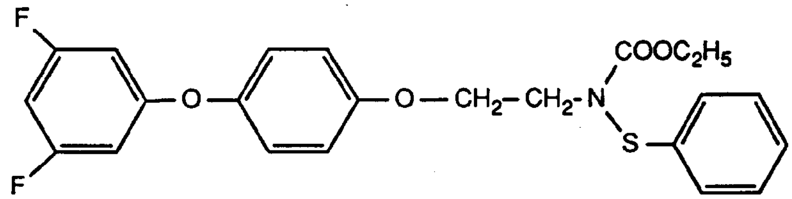

,

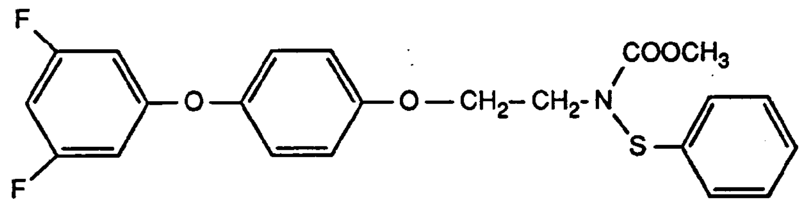

,

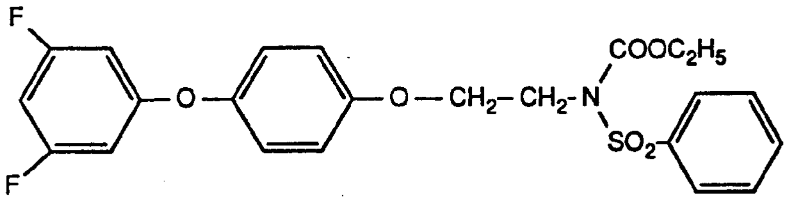

,

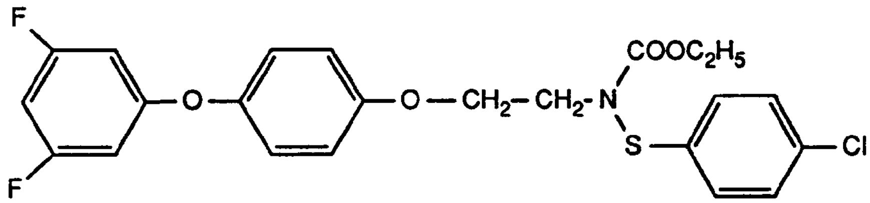

und

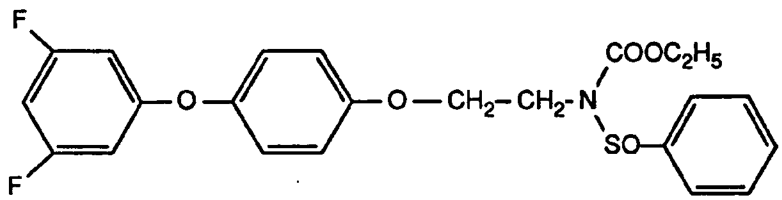

.

4. Mittel gemäss Anspruch 1, enthaltend die Verbindung

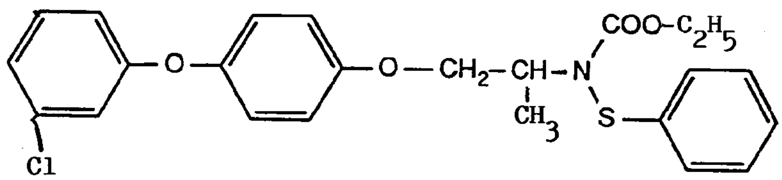

,

5. Verfahren zur Herstellung der Verbindungen der Formel I gemäss Anspruch 1, dadurch gekennzeichnet, dass man entweder

a) ein Phenoxyphenol der Formel

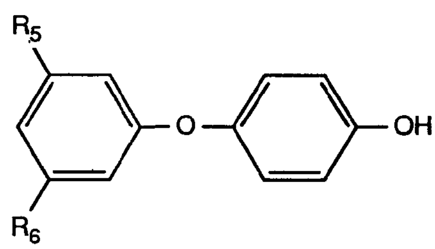

(II),

worin $R_5$ und $R_6$ die unter Formel I gegebenen Bedeutungen haben, in Gegenwart einer Base mit einem Aethylcarbaminsäure-Derivat der Formel

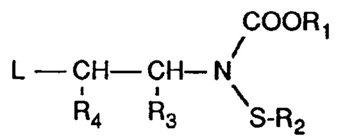

(III),

worin $R_1$, $R_2$, $R_3$ und $R_4$ die unter Formel I gegebenen Bedeutungen haben und L für eine Abgangsgruppe steht, umsetzt und das erhaltene Produkt der Formel

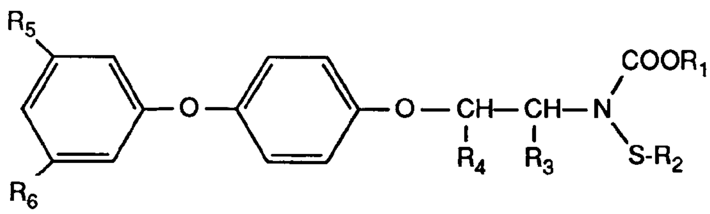

(Ia),

worin $R_1$, $R_2$, $R_3$, $R_4$, $R_5$ und $R_6$ die unter Formel I gegebenen Bedeutungen haben, gewünschtenfalls zu den Verbindungen der Formel I, worin n eins oder zwei bedeutet, oxidiert, oder
b) ein Aethylcarbaminsäure-Derivat der Formel

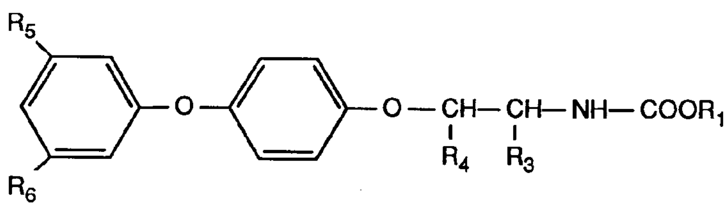

(IV)

worin $R_1$, $R_3$, $R_4$, $R_5$ und $R_5$ die unter Formel I gegebenen Bedeutungen haben, in Gegenwart einer Base mit einem Sulfensäurehalogenid der Formel

Hal-S-$R_2$ (V),

worin $R_2$ die unter Formel I gegebene Bedeutung hat, und Hal für Halogen steht, umsetzt und das erhaltene Produkt der Formel Ia gewünschtenfalls zu den Verbindungen der Formel I, worin n eins oder zwei bedeutet, oxidiert; oder
c) ein Aethylcarbaminsäure-Derivat der Formel IV in Gegenwart einer Base mit einem Sulfinsäurehalogenid der Formel

Hal-SO-$R_2$ (VI),

worin $R_2$ die unter Formel I gegebene Bedeutung hat, und Hal für Halogen steht, umsetzt und das erhaltene Produkt der Formel

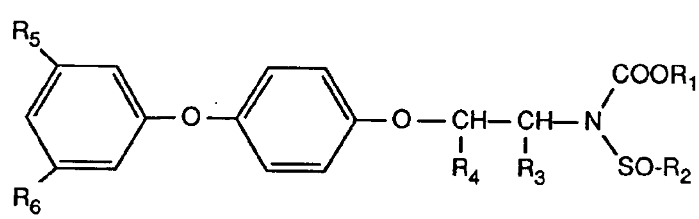

(Ib),

worin $R_1$, $R_2$, $R_3$, $R_4$, $R_5$ und $R_6$ die unter Formel I gegebenen Bedeutungen haben, gewünschtenfalls zu den Verbindungen der Formel I, worin n zwei bedeutet, oxidiert; oder
d) ein Aethylcarbaminsäure-Derivat der Formel IV in Gegenwart einer Base mit einem Sulfonsäurehalogenid der Formel

Hal-$SO_2$-$R_2$ (VII),

worin $R_2$ die unter Formel I gegebene Bedeutung hat und Hal für Halogen steht, umsetzt zu einer Verbindung der Formel

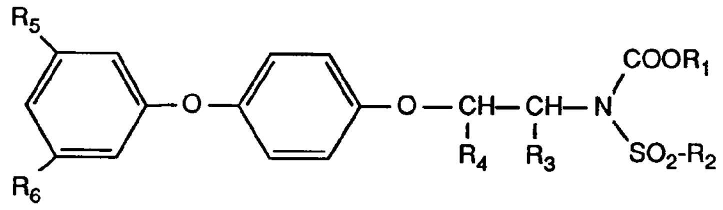

(Ic),

worin $R_1$, $R_2$, $R_3$, $R_4$, $R_5$ und $R_6$ die unter Formel I gegebenen Bedeutungen haben; oder

e) ein Aethylaminderivat der Formel

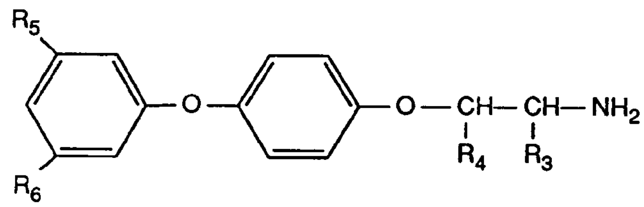

(VIII),

worin $R_3$, $R_4$, $R_5$ und $R_6$ die unter Formel I gegebenen Bedeutungen haben, in Gegenwart einer Base mit einem Halogenameisensäurederivat der Formel

Hal-$COOR_1$ (IX),

worin $R_1$ die unter Formel I gegebene Bedeutung hat und Hal für Halogen, steht, acyliert und das erhaltene Zwischenprodukt der Formel IV in Gegenwart einer Base mit einem Sulfensäurehalogenid der Formel V sulfenyliert und das erhaltene Produkt der Formel Ia gewünschtenfalls zu den Verbindungen der Formel I, worin n eins oder zwei bedeutet, oxidiert; oder

f) ein Aethylaminderivat der Formel VIII in Gegenwart einer Base mit einem Sulfensäurehalogenid der Formel V sulfeniert und das erhaltene Zwischenprodukt der Formel

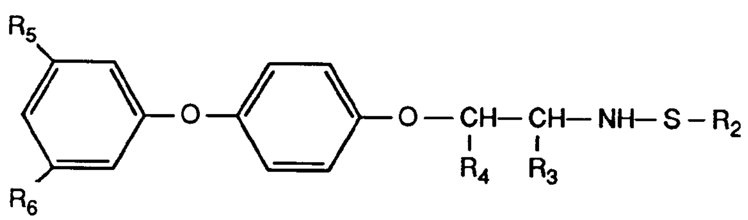

(X),

worin $R_2$, $R_3$, $R_4$, $R_5$ und $R_6$ die unter Formel I gegebenen Bedeutungen haben, in Gegenwart einer Base mit einem Halogenameisensäurederivat der Formel IX acyliert und das erhaltene Produkt der Formel Ia gewünschtenfalls zu den Verbindungen der Formel I, worin n eins oder zwei bedeutet, oxidiert; oder

g) zur Herstellung einer Verbindung der Formel Ic ein Alkalisalz einer Verbindung der Formel

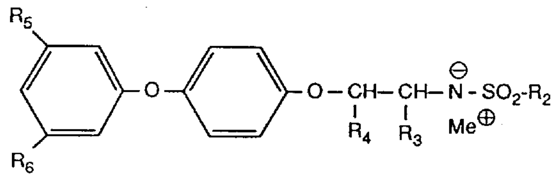

(Xa)

mit einer Verbindung der Formel IX umsetzt, wobei $R_1$ bis $R_6$ die unter Formel I angegebenen Bedeutungen haben und $Me^{\oplus}$ ein Alkalimetallkation bedeutet.

6. Verwendung einer Verbindung der Formel I gemäss Anspruch 1 zur Bekämpfung von Schädlingen an Pflanzen und Tieren, im Vorratsschutz und in der Hygiene.

7. Verwendung gemäss Anspruch 6 zur Bekämpfung von Arthropoden, insbesondere von Insekten und Arachniden.

8. Verwendung gemäss Anspruch 7 zur Bekämpfung von Larven und Eiern phytophager Schadinsekten und -milben.

9. Verwendung gemäss Anspruch 7 zur Abtötung von Eiern pflanzenschädigender Insekten und Ektoparasiten.

10. Verwendung gemäss Anspruch 7 zur Hemmung der Entwicklung von Larven pflanzenschädigender Insekten und Ektoparasiten.

## Claims

**Claims for the following Contracting States : AT, BE, CH, DE, DK, FR, GB, IT, LI, NL**

1. A 2-phenoxyphenoxyethylcarbamic ester of the formula

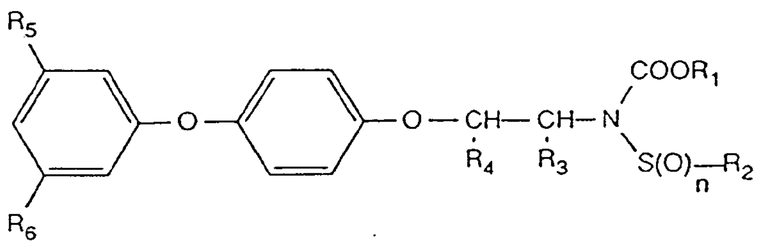

(I)

where $R_1$ is $C_1$-$C_4$alkyl or $C_3$-$C_4$alkenyl, $R_2$ is $C_1$-$C_4$alkyl or a radical of the formula

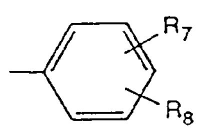

$R_3$ is hydrogen or methyl, $R_4$ is hydrogen, $R_5$ and $R_6$ are either both fluorine, or $R_6$ is chlorine and $R_6$ is hydrogen, $R_7$ and $R_8$ independently of one another are hydrogen, halogen or $C_1$-$C_4$alkyl and n is zero, one or two.

2. A compound according to claim 1, selected from the group comprising

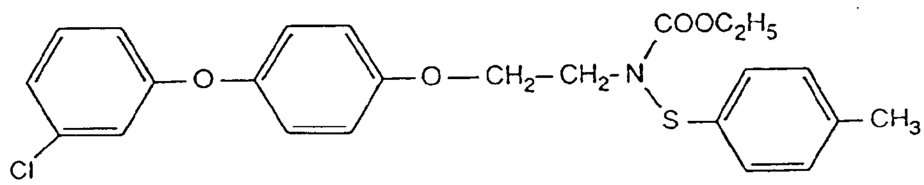

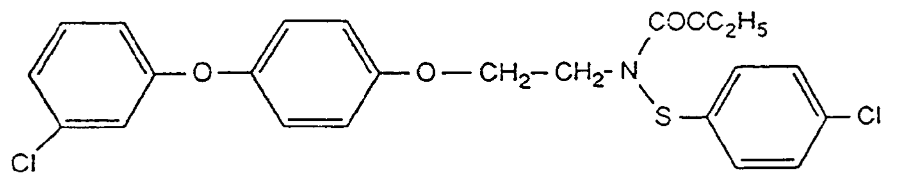

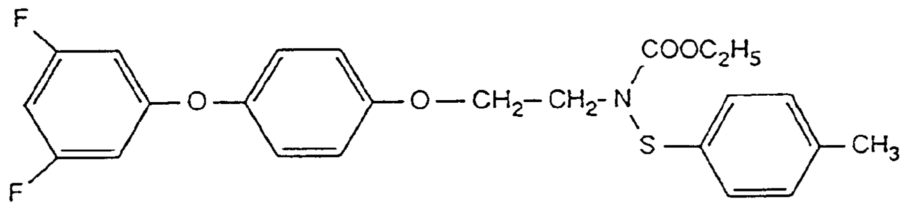

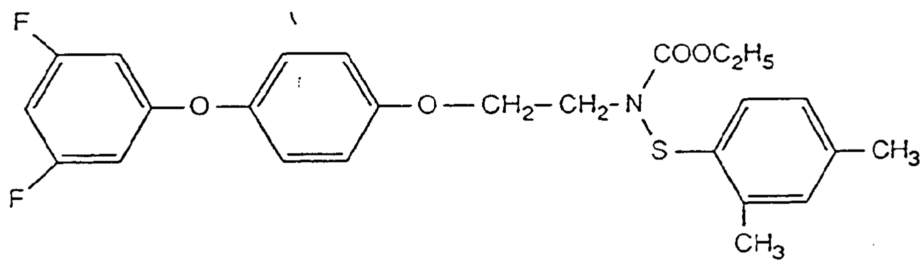

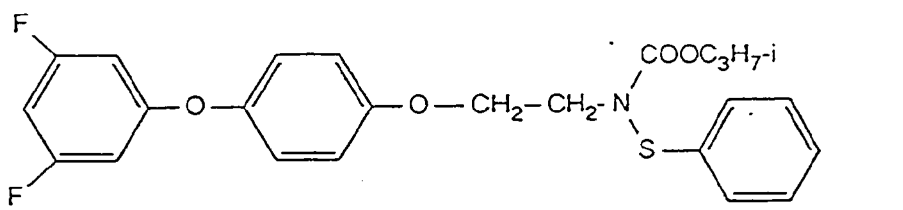

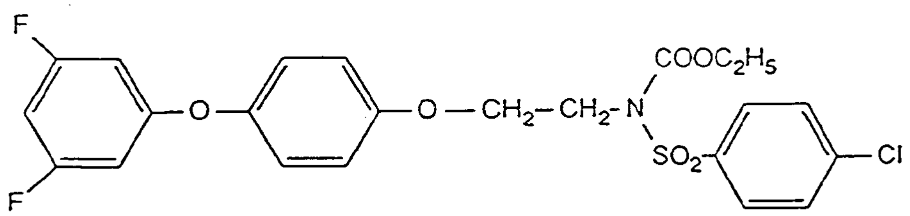

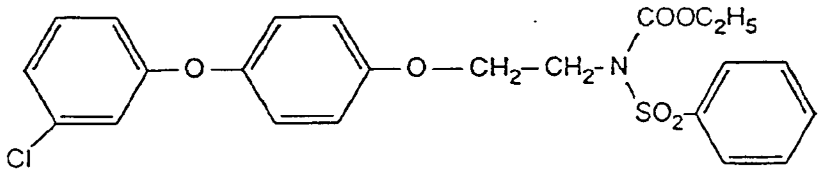

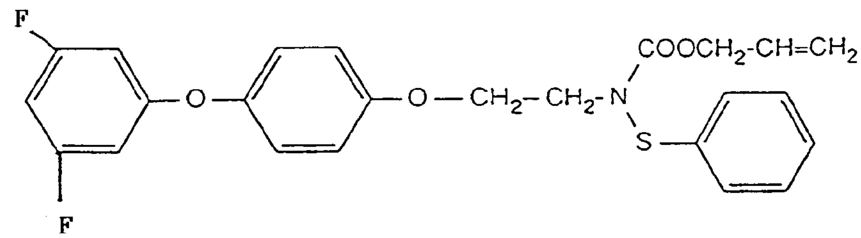

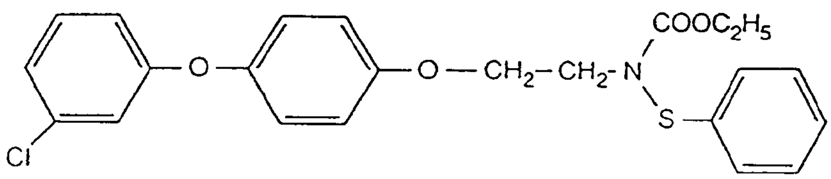

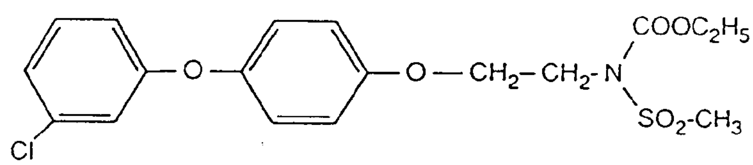

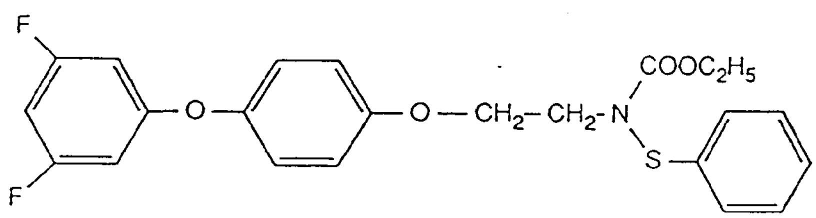

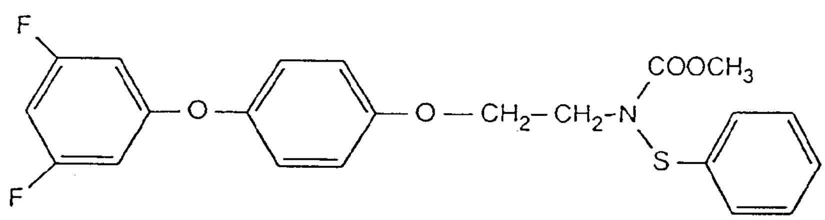

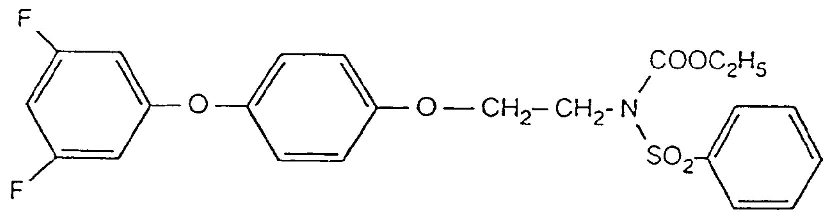

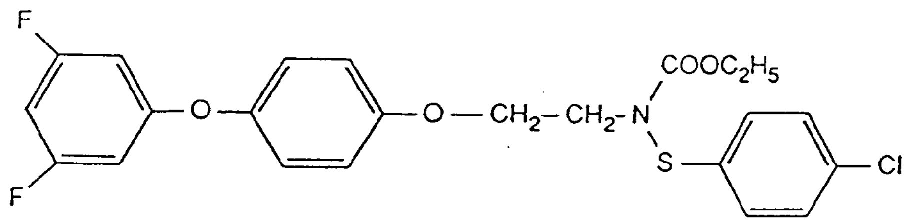

and

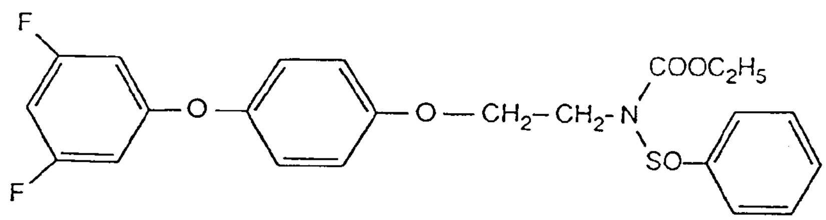

3. A compound of the formula

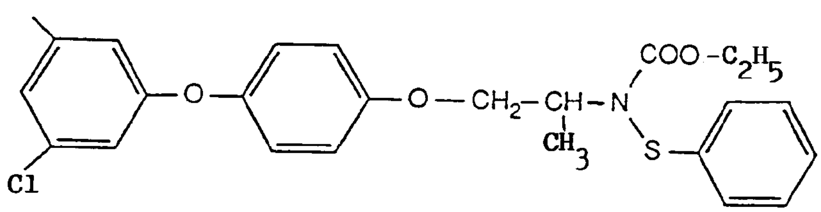

,

according to claim 1.

4. A process for the preparation of a compound of the formula I according to claim 1, wherein either

a) a phenoxyphenol of the formula

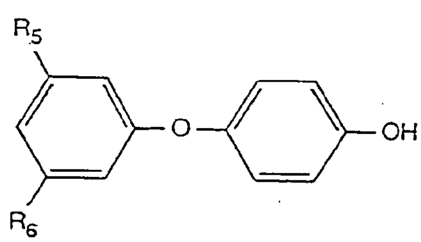

(II)

where $R_5$ and $R_5$ are as defined under formula I is reacted, in the presence of a base, with an ethylcarbamic acid derivative of the formula

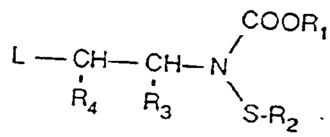

(III)

where $R_1$, $R_2$, $R_3$ and $R_4$ are as defined under formula I and L is a leaving group, and the resulting product of the formula

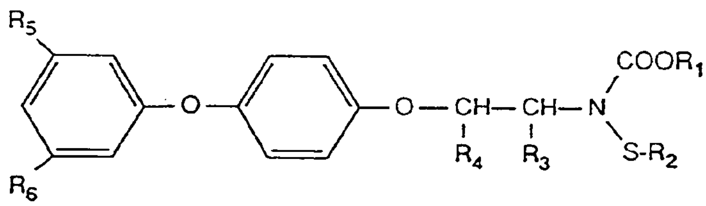

(Ia)

where $R_1$, $R_2$, $R_3$, $R_4$, $R_5$ and $R_5$ are as defined under formula I, may be oxidized to give a compound of the formula I where n is one or two, or

b) an ethylcarbamic acid derivative of the formula

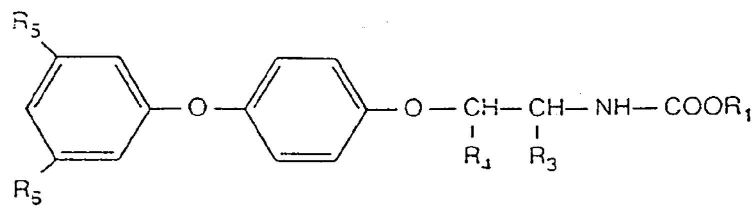

(IV)

where $R_1$, $R_3$, $R_4$, $R_5$ and $R_6$ are as defined under formula I, is reacted, in the presence of a base, with a sulfenyl halide of the formula

$$Hal\text{-}S\text{-}R_2 \qquad (V)$$

where $R_2$ is as defined under formula I and Hal is halogen, and the resulting product of the formula Ia may be oxidized to give a compound of the formula I where n is one or two; or

c) an ethylcarbamic acid derivative of the formula IV is reacted, in the presence of a base, with a sulfinyl halide of the formula

$$Hal\text{-}SO\text{-}R_2 \qquad (VI)$$

where $R_2$ is as defined under formula I and Hal is halogen, and the resulting product of the formula

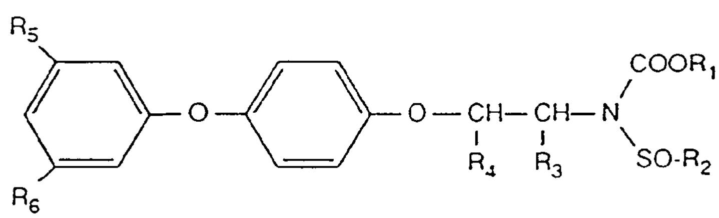

(Ib)

where $R_1$, $R_2$, $R_3$, $R_4$, $R_5$ and $R_5$ are as defined under formula I may be oxidized to give a compound of the formula I where n is two; or

d) an ethylcarbamic acid derivative of the formula IV is reacted, in the presence of a base, with a sulfonyl halide of the formula

$$Hal\text{-}SO_2\text{-}R_2 \qquad (VII)$$

where $R_2$ is as defined under formula I and Hal is halogen, to give a compound of the formula

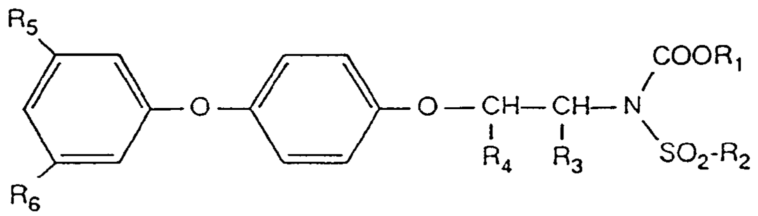

(Ic)

where $R_1$, $R_2$, $R_3$, $R_4$, $R_5$ and $R_5$ are as defined under formula I; or

e) an ethylamine derivative of the formula

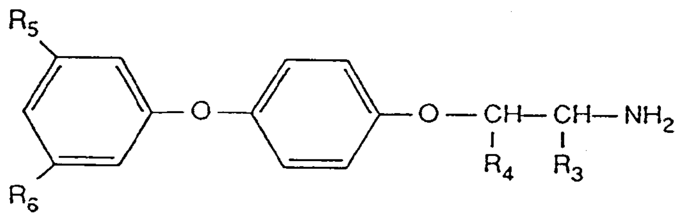

(VIII)

where $R_3$, $R_4$, $R_5$ and $R_5$ are as defined under formula I is acylated, in the presence of a base, with a haloformic acid derivative of the formula

$$Hal\text{-}COOR_1 \qquad (IX)$$

where $R_1$ is as defined under formula I and Hal is halogen, and the resulting intermediate of the formula IV is sulfenylated with a sulfenic acid halide of the formula V in the presence of a base, and the resulting product of the formula Ia may be oxidized to give a compound of the formula I where n is one or two; or

f) an ethylamine derivative of the formula VIII is sulfenated with a sulfenic acid halide of the formula V in the presence of a base and the resulting intermediate of the formula

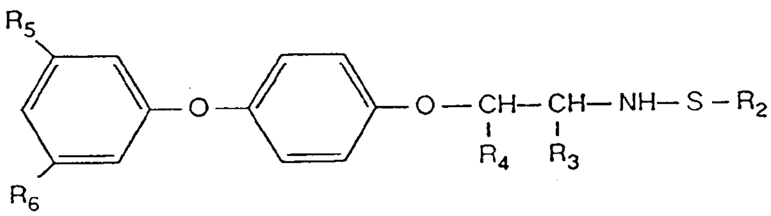

(X)

where $R_2$, $R_3$, $R_4$, $R_5$ and $R_6$ are as defined under formula I is acylated with a haloformic acid derivative of the formula IX in the presence of a base, and the resulting product of the formula Ia may be oxidized to give a compound of the formula I where n is one or two; or

g) for the preparation of a compound of the formula Ic, an alkali metal salt of a compound of the formula

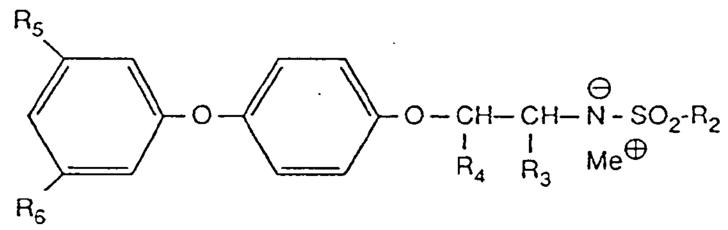 (Xa)

is reacted with a compound of the formula IX, $R_1$ to $R_6$ being as defined under formula I and $Me^{\oplus}$ being an alkali metal cation.

5. A pesticidal composition containing, as the active component, at least one compound of the formula I according to claim 1.

6. A composition according to claim 5, which contains additionally at least one carrier.

7. Use of a compound of the formula I according to claim 1 for controlling pests on plants and animals, in the protection of stored goods, and in hygiene.

8. Use according to claim 7 for controlling arthropods, in particular insects and arachnids.

9. Use according to claim 8 for controlling larvae and eggs of phytophagous harmful insects and harmful mites.

10. Use according to claim 8 for destroying eggs of insects and ectoparasites which damage plants.

11. Use according to claim 8 for inhibiting the development of larvae of insects and ectoparasites which damage plants.

**Claims for the following Contracting State : ES**

1. A pesticidal composition, containing, as the active component at least one 2-phenoxyphenoxyethylcarbamic ester of the formula

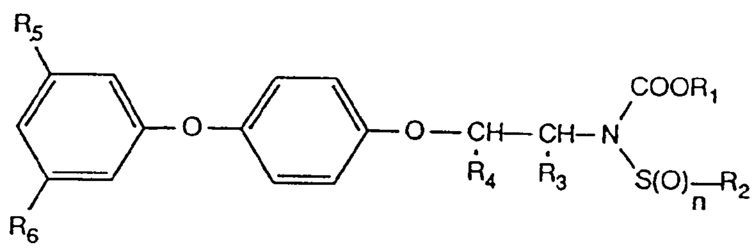 (I)

where $R_1$ is $C_1$-$C_4$alkyl or $C_3$-$C_4$alkenyl, $R_2$ is $C_1$-$C_4$alkyl or a radical of the formula

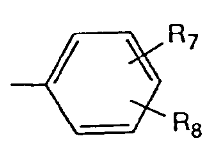

$R_3$ is hydrogen or methyl, $R_4$ is hydrogen, $R_5$ and $R_6$ are either both fluorine, or $R_6$ is chlorine and $R_6$ is hydrogen, $R_7$ and $R_8$ independently of one another are hydrogen, halogen or $C_1$-$C_4$alkyl and n is zero, one or two.

2. A composition according to claim 1, which contains additionally at least one carrier.

3. A composition according to claim 1, containing an active substance selected from the group comprising

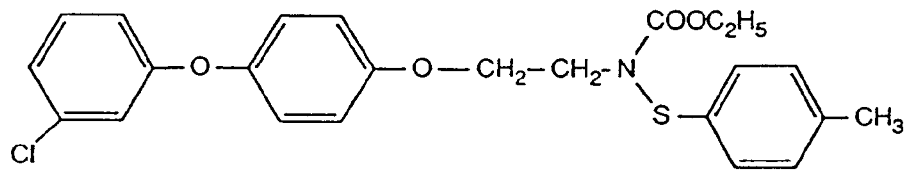

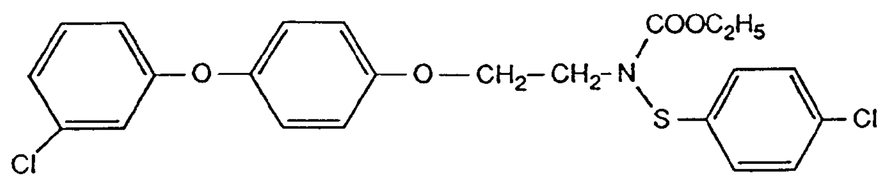

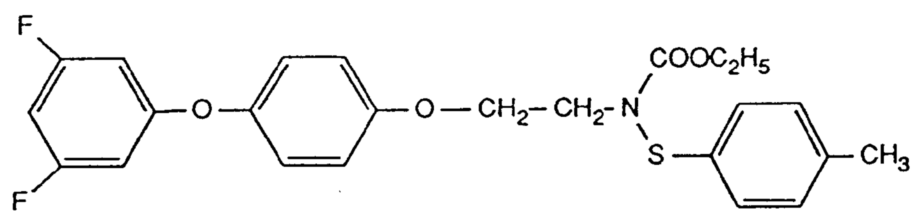

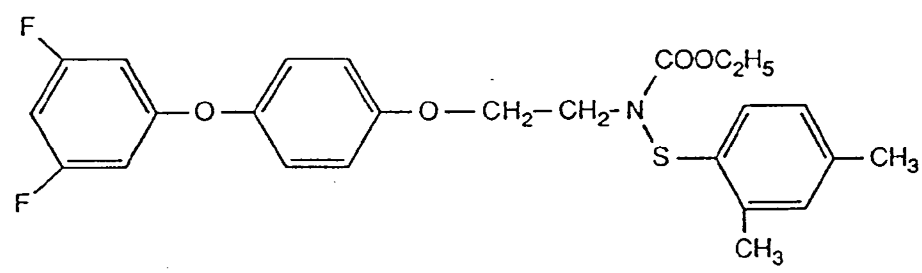

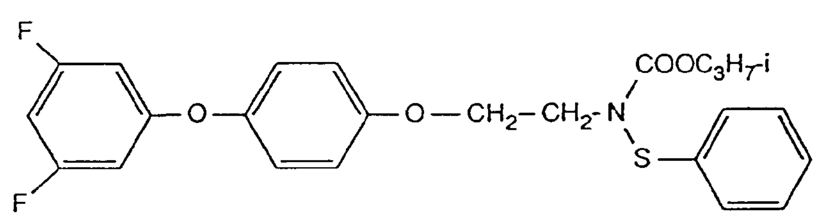

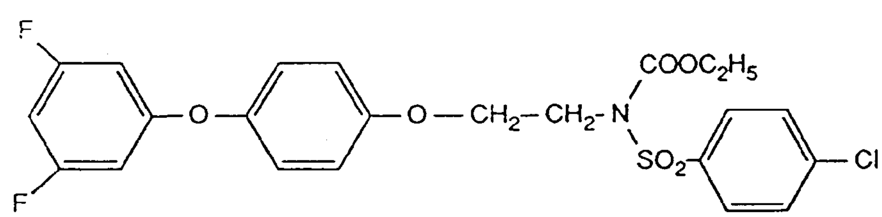

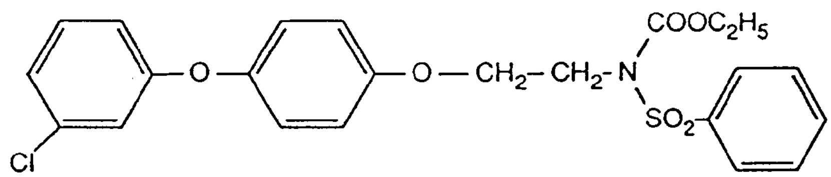

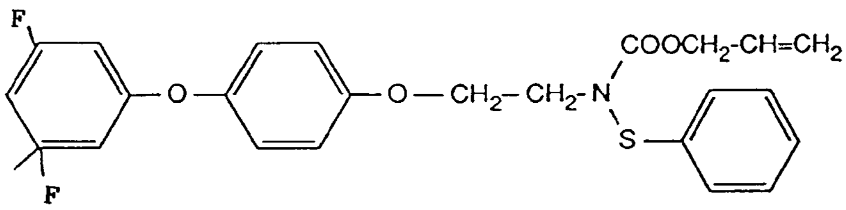

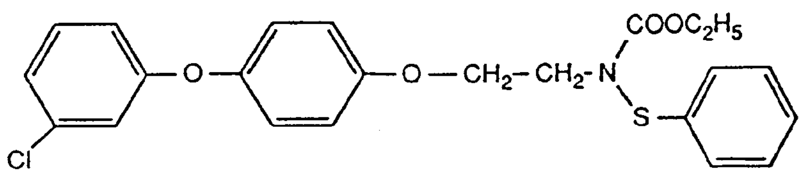

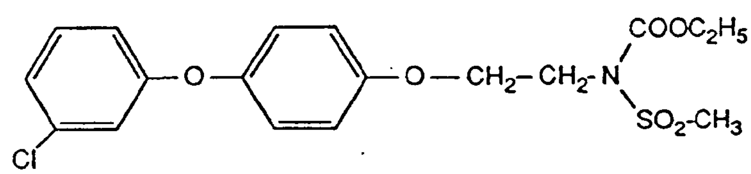

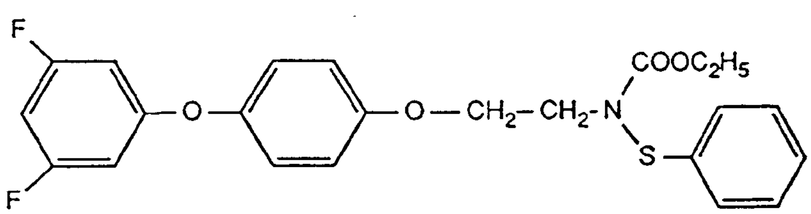

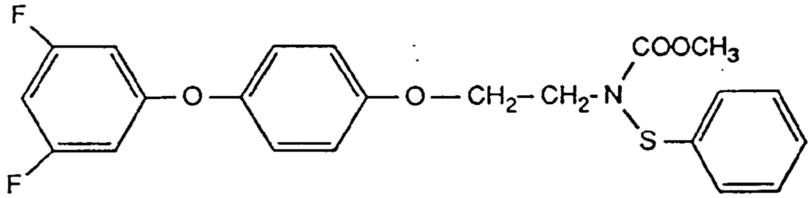

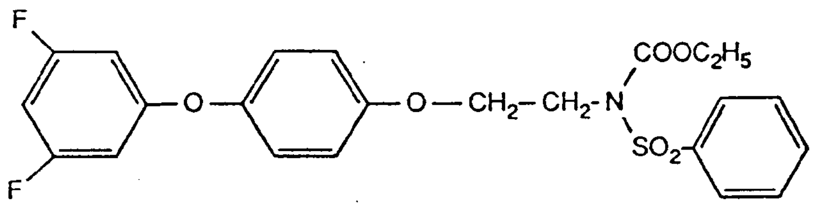

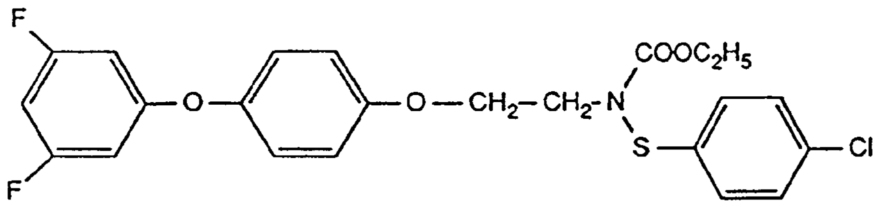

and

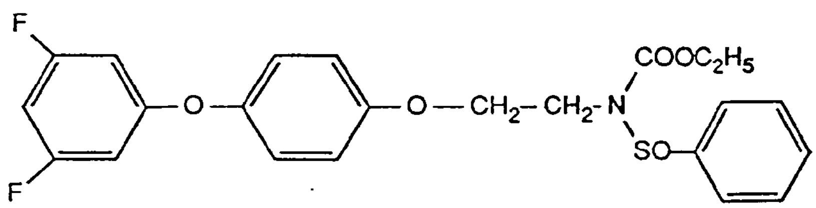

4. A composition according to claim 1, containing the compound

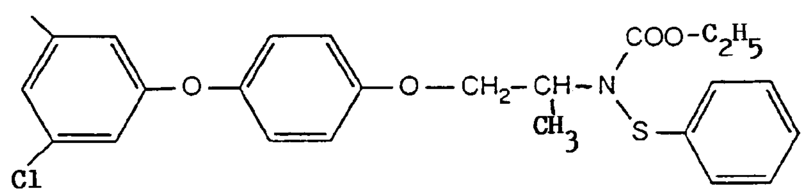

,

5. A process for the preparation of a compound of the formula I acccording to claim 1, wherein either
a) a phenoxyphenol of the formula

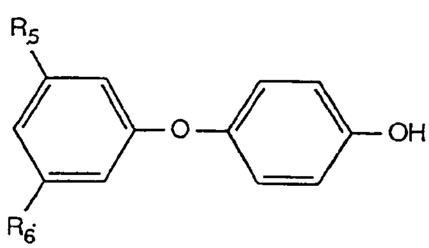

(II)

where $R_5$ and $R_5$ are as defined under formula I is reacted, in the presence of a base, with an ethylcarbamic acid derivative of the formula

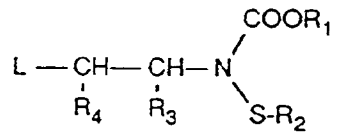

(III)

where $R_1$, $R_2$, $R_3$ and $R_4$ are as defined under formula I and L is a leaving group, and the resulting product of the formula

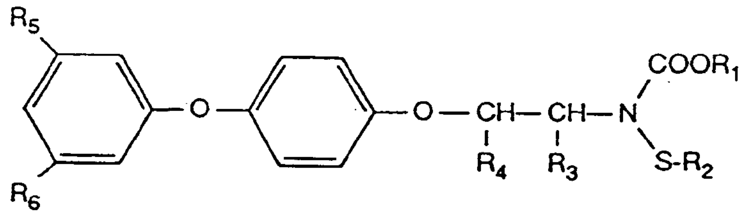

(Ia)

where $R_1$, $R_2$, $R_3$, $R_4$, $R_5$ and $R_6$ are as defined under formula I, may be oxidized to give a compound of the formula I where n is one or two, or

b) an ethylcarbamic acid derivative of the formula

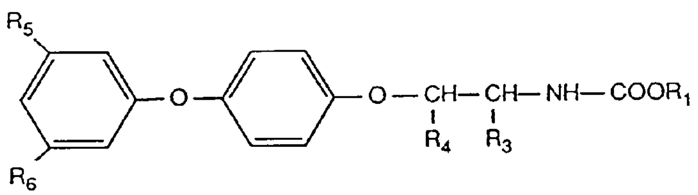

(IV)

where $R_1$, $R_3$, $R_4$, $R_6$ and $R_6$ are as defined under formula I, is reacted, in the presence of a base, with a sulfenyl halide of the formula

Hal-S-$R_2$ (V)

where $R_2$ is as defined under formula I and Hal is halogen, and the resulting product of the formula Ia may be oxidized to give a compound of the formula I where n is one or two; or

c) an ethylcarbamic acid derivative of the formula IV is reacted, in the presence of a base, with a sulfinyl halide of the formula

Hal-SO-$R_2$ (VI)

where $R_2$ is as defined under formula I and Hal is halogen, and the resulting product of the formula

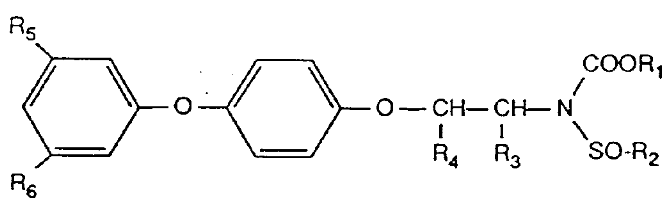

(Ib)

where $R_1$, $R_2$, $R_3$, $R_4$, $R_6$ and $R_6$ are as defined under formula I may be oxidized to give a compound of the formula I where n is two; or

d) an ethylcarbamic acid derivative of the formula IV is reacted, in the presence of a base, with a sulfonyl halide of the formula

Hal-$SO_2$-$R_2$ (VII)

where $R_2$ is as defined under formula I and Hal is halogen, to give a compound of the formula

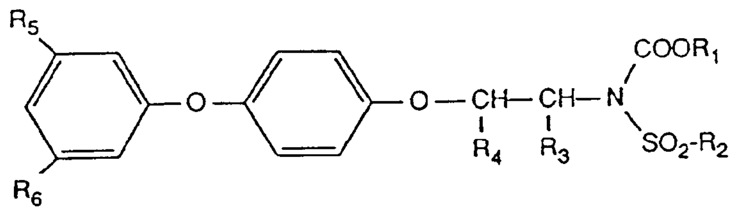

(Ic)

where $R_1$, $R_2$, $R_3$, $R_4$, $R_5$ and $R_5$ are as defined under formula I; or

e) an ethylamine derivative of the formula

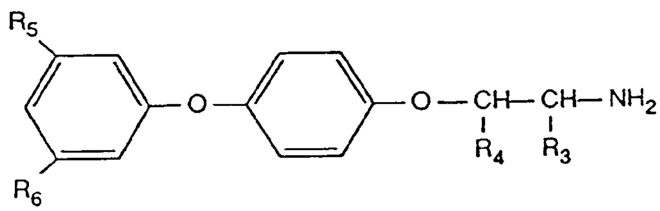

(VIII)

where $R_3$, $R_4$, $R_5$ and $R_5$ are as defined under formula I is acylated, in the presence of a base, with a haloformic acid derivative of the formula

Hal-$COOR_1$ (IX)

where $R_1$ is as defined under formula I and Hal is halogen, and the resulting intermediate of the formula IV is sulfenylated with a sulfenic acid halide of the formula V in the presence of a base, and the resulting product of the formula Ia may be oxidized to give a compound of the formula I where n is one or two; or

f) an ethylamine derivative of the formula VIII is sulfenated with a sulfenic acid halide of the formula V in the presence of a base and the resulting intermediate of the formula

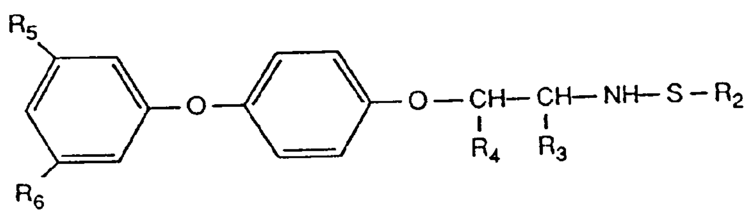

(X)

where $R_2$, $R_3$, $R_4$, $R_5$ and $R_6$ are as defined under formula I is acylated with a haloformic acid derivative of the formula IX in the presence of a base, and the resulting product of the formula Ia may be oxidized to give a compound of the formula I where n is one or two; or

g) for the preparation of a compound of the formula Ic, an alkali metal salt of a compound of the formula

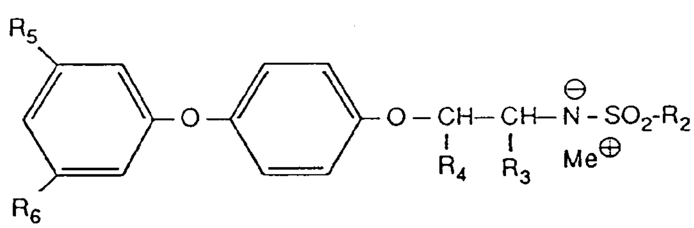

(Xa)

is reacted with a compound of the formula IX, $R_1$ to $R_6$ being as defined under formula I and $Me^{\oplus}$ being an alkali metal cation.

6. Use of a compound of the formula I according to claim 1 for controlling pests on plants and animals, in the protection of stored goods, and in hygiene.

7. Use according to claim 6 for controlling arthropods, in particular insects and arachnids.

8. Use according to claim 7 for controlling larvae and eggs of phytophagous harmful insects and harmful mites.

9. Use according to claim 7 for destroying eggs of insects and ectoparasites which damage plants.

10. Use according to claim 7 for inhibiting the development of larvae of insects and ectoparasites which damage plants.

## Revendications

**Revendications pour les Etats contractants suivants : AT, BE, CH, DE, DK, FR, GB, IT, LI, NL**

1. 2-phénoxyphénoxyéthylcarbamate de formule

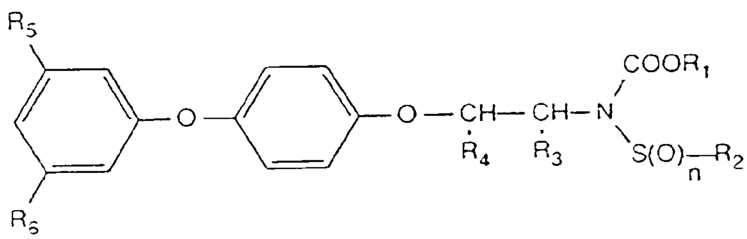

(I),

dans laquelle
$R_1$ représente un alkyle $C_1$-$C_4$ ou alcényle $C_3$-$C_4$
$R_2$ un alkyle $C_1$-$C_4$ ou un reste de formule

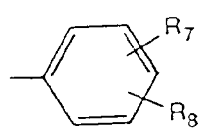

,

$R_3$ l'hydrogène ou le méthyle, $R_4$ l'hydrogène,
$R_5$ et $R_6$ représentent soit tous les deux le fluor soit $R_6$ le chlore et $R_6$ l'hydrogène,
$R_7$ et $R_8$ représentent indépendamment l'un de l'autre l'hydrogène, un halogène ou alkyle $C_1$-$C_4$ et n est zéro, un ou deux.

2. Composé selon la revendication 1, choisi dans le groupe constitué de

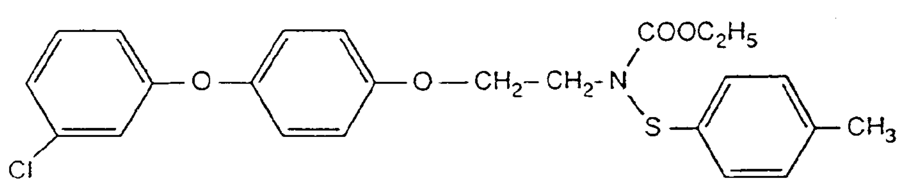

.

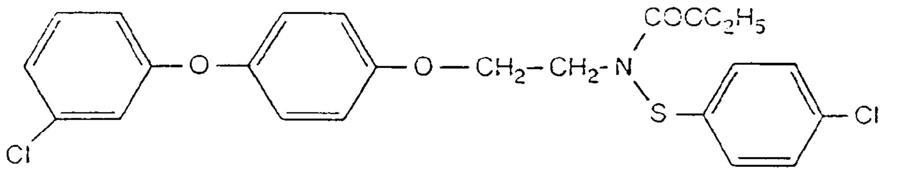

,

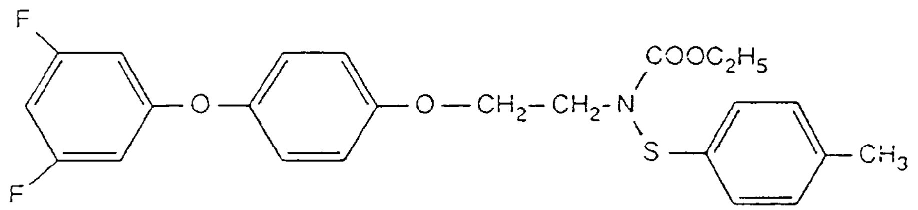

,

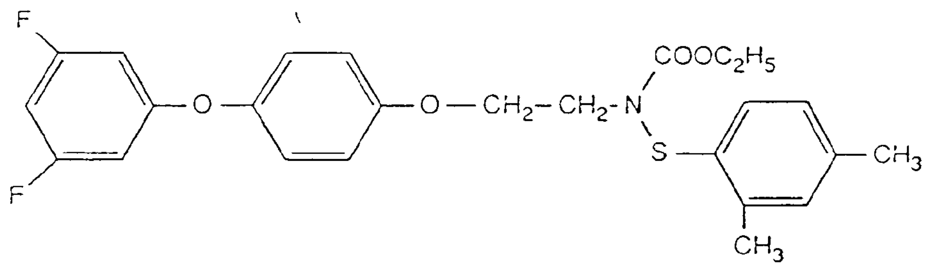

,

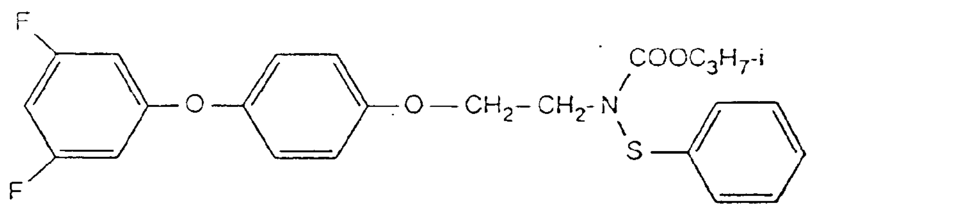

,

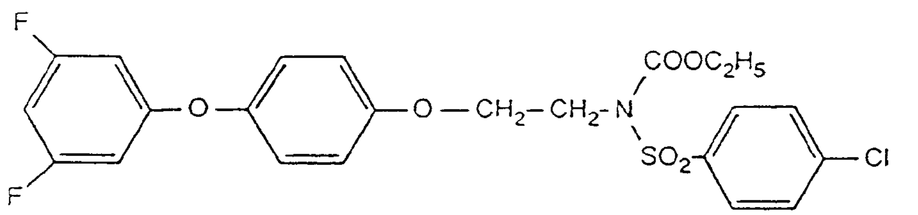

,

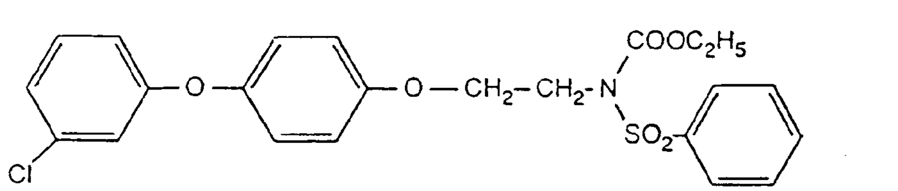

,

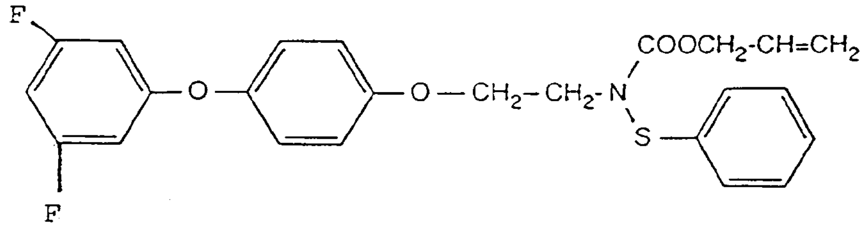

,

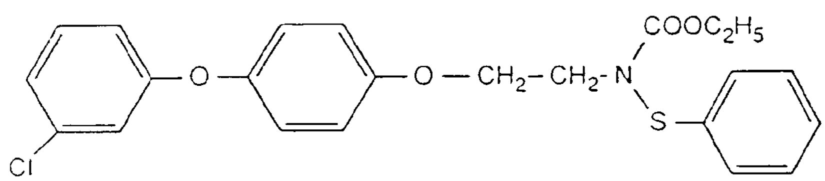

,

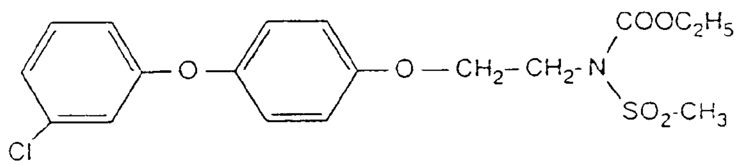

,

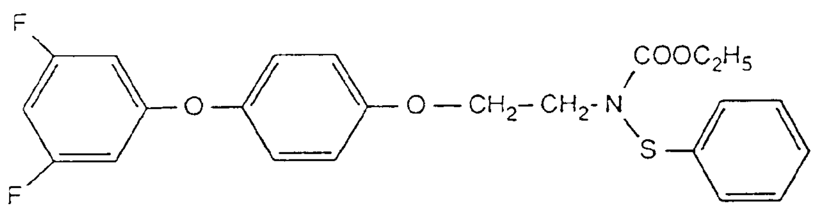

,

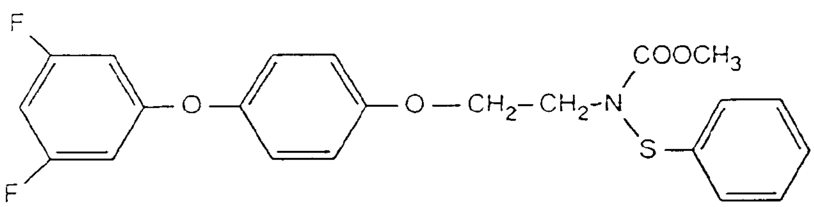

,

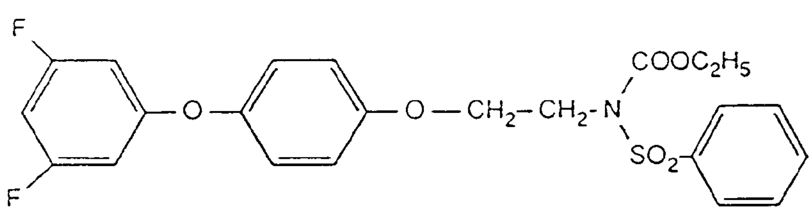

,

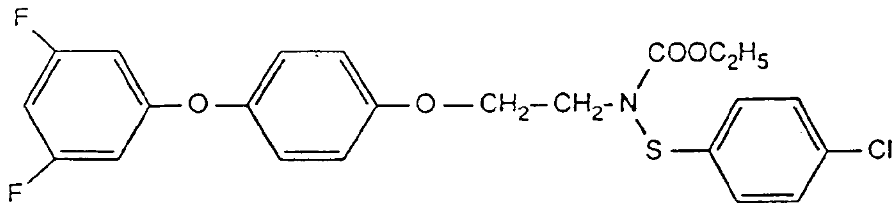

et

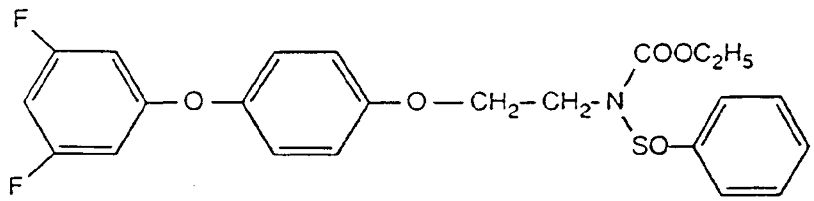

3. Composé de formule

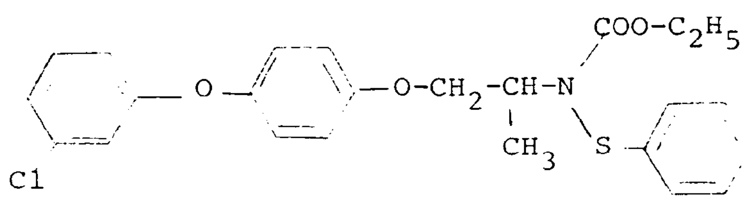

selon la revendication 1.

4. Procédé de préparation d'un composé de formule I selon la revendication 1 caractérisé en ce qu'on procède

a) en faisant réagir un phénoxyphénol de formule

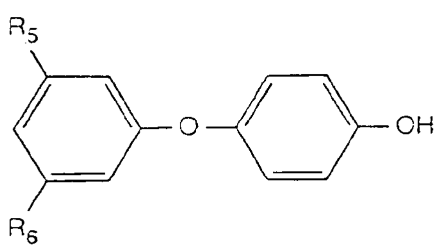

(II),

dans laquelle $R_5$ et $R_6$ ont la signification donnée pour la formule I, en présence d'une base avec un dérivé d'acide éthylcarbamique de formule

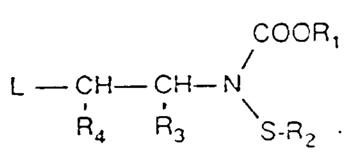

(III),

dans laquelle $R_1$, $R_2$, $R_3$ et $R_4$ ont les significations données pour la formule I et L est un groupe labile,

et en oxydant le produit obtenu de formule

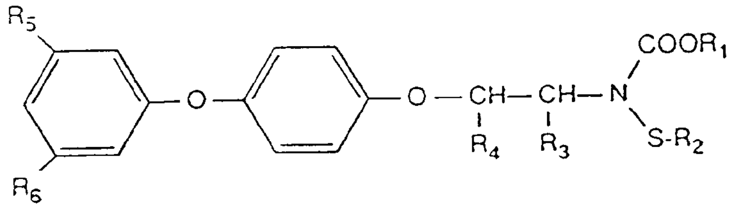
(Ia),

dans laquelle $R_1$, $R_2$, $R_3$, $R_4$, $R_5$ et $R_6$ ont les significations données pour la formule I, si c'est souhaité en un composé de formule I, dans laquelle n représente un ou deux; ou
b) en faisant réagir un dérivé d'acide éthylcarbamique de formule

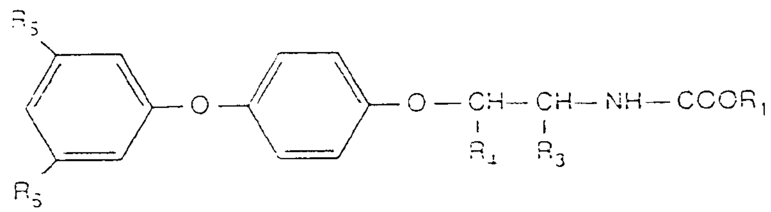
(IV),

dans laquelle $R_1$, $R_3$, $R_4$, $R_5$ et $R_6$ ont les significations données pour la formule I, en présence d'une base avec un halogénure d'acide sulfénique de formule

Hal-S-$R_2$ (V),

dans laquelle $R_2$ a la signification donnée pour la formule I, et Hal représente un halogène, et on oxvde le produit obtenu de formule Ia si c'est souhaité en un composé de formule I dans lequel n représente un ou deux, ou
c) en faisant réagir un dérivé d'acide éthylcarbamique de formule IV en présence d'une base avec un halogénure d'acide sulfinique de formule

Hal-SO-$R_2$ (VI),

dans laquelle $R_2$ a la signification donnée à la formule I et Hal représente un halogène, et on oxyde le produit obtenu de formule

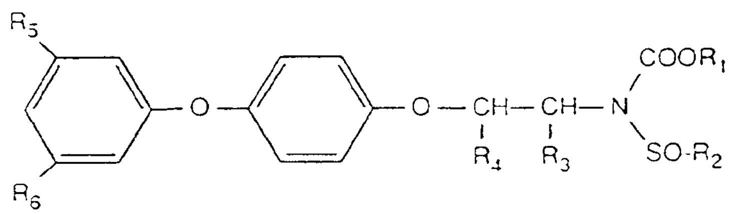
(Ib),

dans laquelle $R_1$, $R_2$, $R_3$, $R_4$, $R_6$ et $R_6$ ont les significations données pour la formule I, le cas échéant pour donner un composé de formule I dans laquelle n représente deux; ou
d) en faisant réagir un dérivé d'acide éthylcarbamique de formule IV en présence d'une base avec un halogénure d'acide sulfonique de formule

Hal-$SO_2$-$R_2$ (VII),

dans laquelle $R_2$ a la signifiation donnée à la formule I et Hal représente un halogène, pour donner un composé de formule

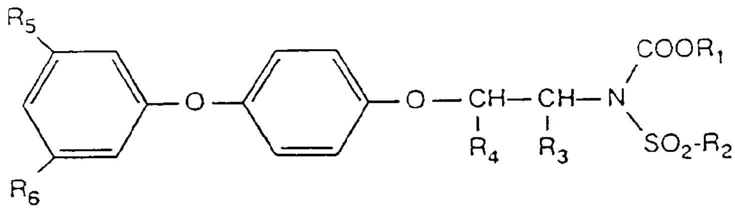

(Ic),

dans laquelle $R_1$, $R_2$, $R_3$, $R_4$, $R_5$ et $R_6$ ont les significations données pour la formule I; ou

e) en acylant un dérivé d'éthylamine de formule

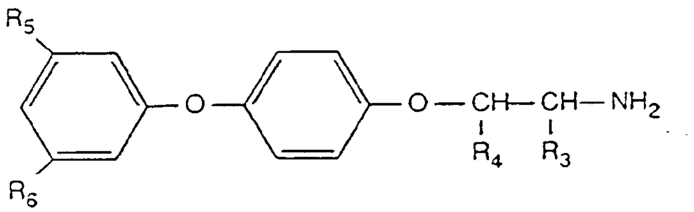

(VIII),

dans laquelle $R_3$, $R_4$, $R_5$ et $R_6$ ont les significations données pour la formule I, en présence d'une base avec un dérivé d'acide halogénoformique de formule

Hal-$COOR_1$ (IX),

dans laquelle $R_1$ a la signification donnée pour la formule I et Hal représente un halogène, et on sulfényle le produit intermédiaire de formule IV en présence d'une base avec un halogénure d'acide sulfénique de formule V et on oxyde le produit obtenu de formule Ia, si c'est souhaité en un composé de formule I, dans lequel n représente un ou deux; ou

f) en sulfénisant un dérivé d'éthylamine de formule VIII en présence d'une base avec un halogénure d'acide sulfénique de formule V, et on acyle le produit intermédiaire obtenu de formule X

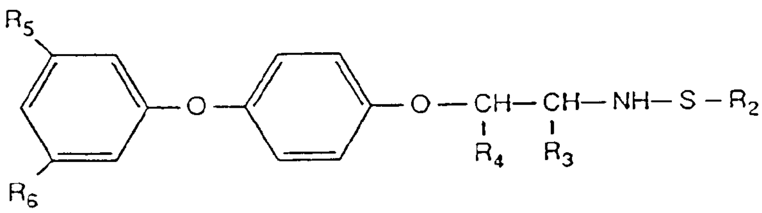

(X),

dans laquelle $R_2$, $R_3$, $R_4$, $R_5$ et $R_5$ ont les significations données pour la formule I, en présence d'une base avec un dérivé d'acide halogénoformique de formule IX et on oxyde le produit obtenu de formule Ia, si c'est souhaité en composé de formule I, dans laquelle n représente un ou deux; ou

g) pour préparer un composé de formule Ic on fait réagir un sel alcalin d'un composé de formule

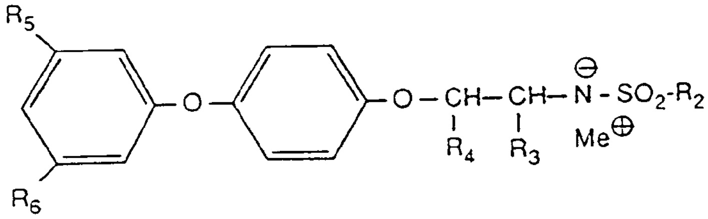

(Xa)

avec un composé de formule IX, $R_1$ à $R_5$ ayant les significations indiquées pour la formule I et $Me^{\oplus}$ est un cation de métal alcalin.

**5.** Moyen de lutte antiparasitaire qui contient comme composant actif au moins un composé de Formule I selon la revendication 1.

6. Moyen selon la revendication 5, caractérisé en ce qu'il contient en plus au moins un véhicule.

7. Utilisation d'un composé de formule I selon la revendication 1 pour combattre les parasites des plantes et des animaux, en protection des réserves et en hygiène.

8. Utilisation selon la revendication 7 pour combattre les arthropodes, notamment les insectes et les arachnides.

9. Utilisation selon la revendication 8 pour combattre les larves et les oeufs d'insectes et de papillons nuisibles phytophages.

10. Utilisation selon la revendication 8 pour tuer les oeufs d'insectes et d'ectoparasites endommageant les plantes.

11. Utilisation selon la revendication 8 pour entraver le développement de larves d'insectes et d'ecto- parasites endommageant les plantes.

**Revendications pour l'Etat contractant suivant : ES**

1. Moyen de lutte antiparasitaire qui contient comme composant actif au moins un 2-phénoxyphénoxycarbamate de formule

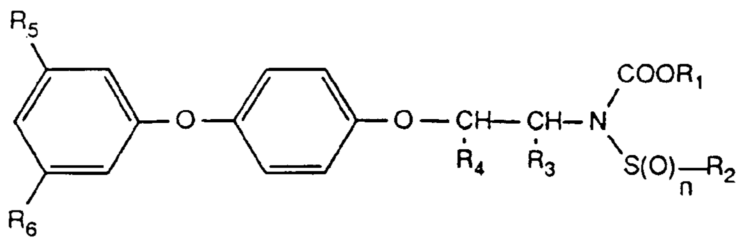

(I),

dans laquelle
$R_1$ représente un alkyle $C_1$-$C_4$ ou alcényle $C_3$-$C_4$,
$R_2$ un alkyle $C_1$-$C_4$ ou un reste de formule

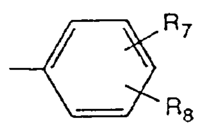

$R_3$ l'hydrogène ou le méthyle, $R_4$ l'hydrogène,
$R_5$ et $R_6$ représentent soit tous les deux le fluor soit $R_6$ le chlore et $R_6$ l'hydrogène,
$R_7$ et $R_8$ représentent indépendamment l'un de l'autre l'hydrogène, un halogène ou alkyle $C_1$-$C_4$ et n est zéro, un ou deux.

2. Moyen selon la revendication 1, caractérisé en ce qu'il contient en plus au moins un véhicule.

3. Moyen selon la revendication 1 qui contient un principe actif choisi dans le groupe

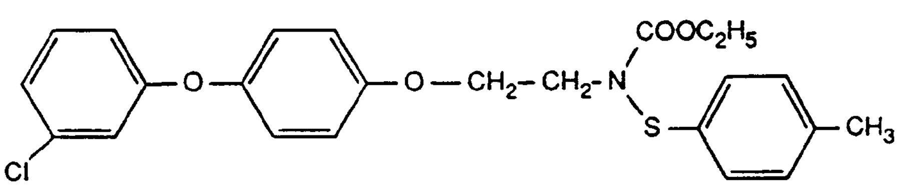

,

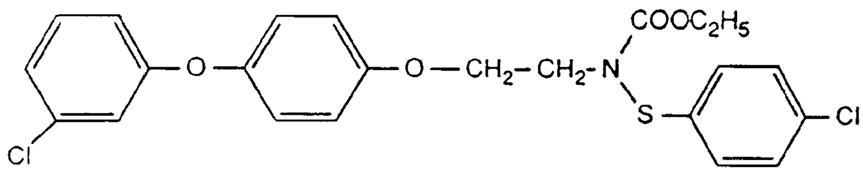

,

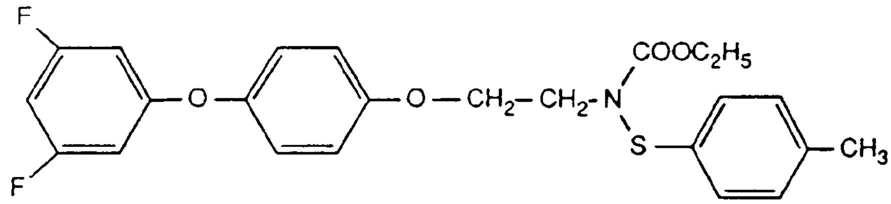

,

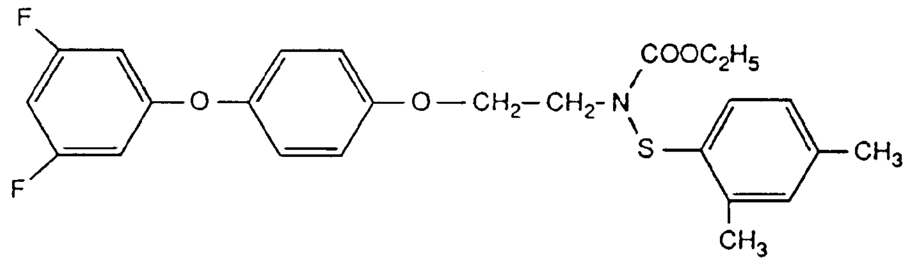

,

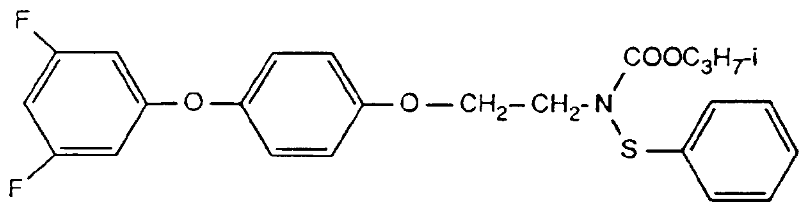

,

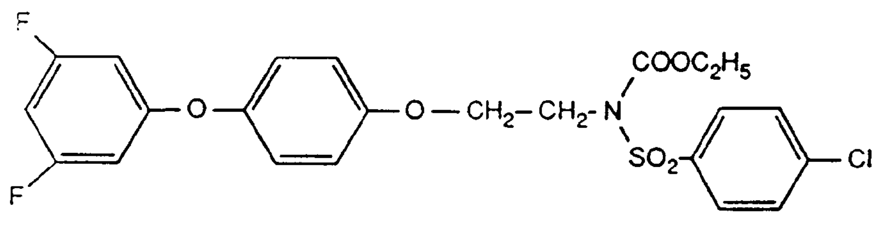

,

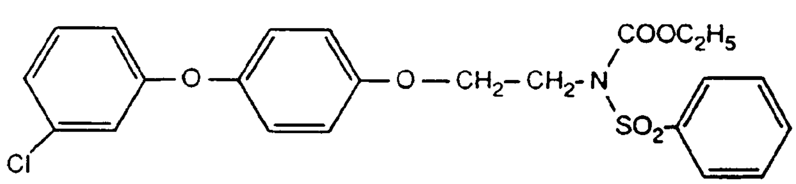

,

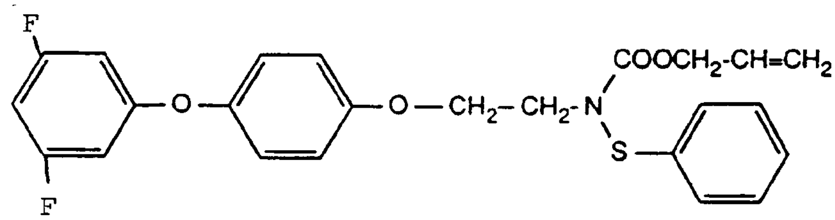

,

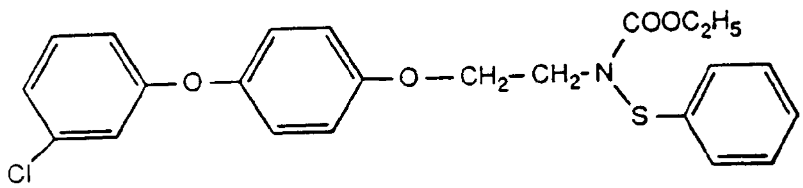

,

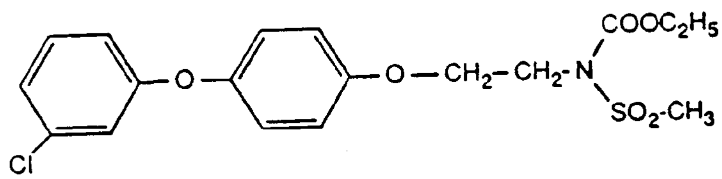

,

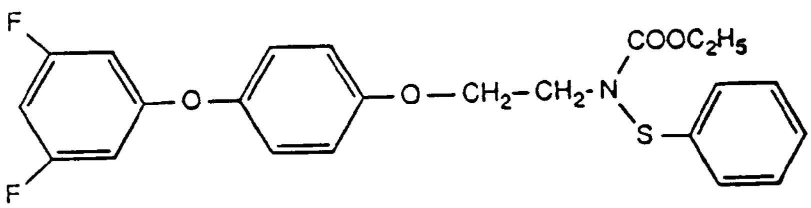

,

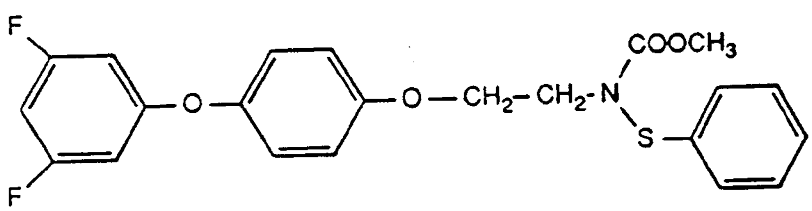

,

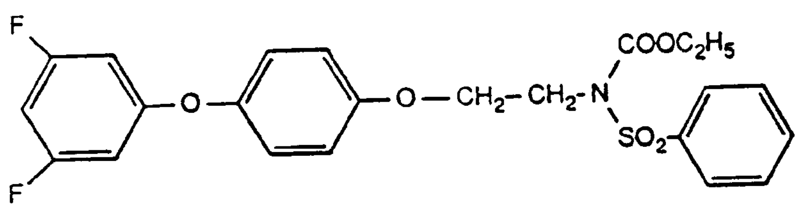

,

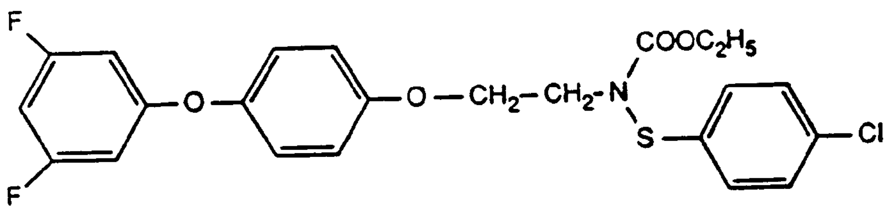

et

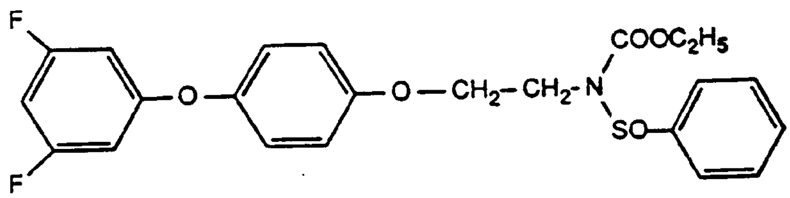

**4.** Moyen selon la revendication 1, qui contient le composé

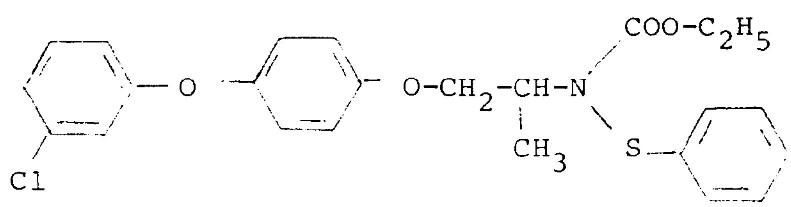

**5.** Procédé de préparation de composés de formule I selon la revendication 1, caractérisé en ce qu'on procède soit

a) en faisant réagir un phénoxyphénol de formule

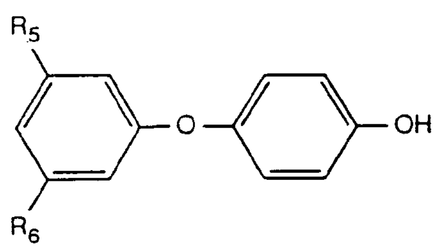

(II),

dans laquelle $R_5$ et $R_6$ ont la signification donnée pour la formule I, en présence d'une base avec un dérivé d'acide éthylcarbamique de formule

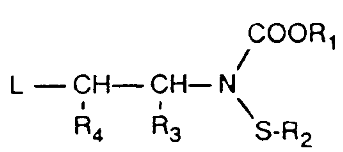

(III),

dans laquelle $R_1$, $R_2$, $R_3$ et $R_4$ ont les significations données pour la formule I et L est un groupe labile, et en oxydant le produit obtenu de formule

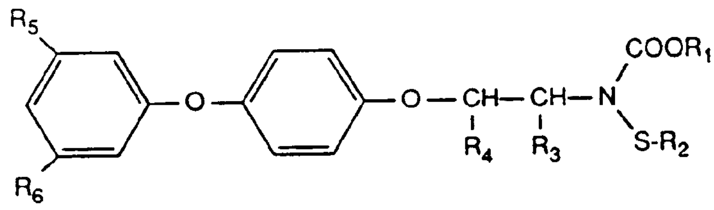

(Ia),

dans laquelle $R_1$, $R_2$, $R_3$, $R_4$, $R_5$ et $R_6$ ont les significations données pour la formule I, si c'est souhaité en un composé de formule I, dans laquelle n représente un ou deux; ou

b) en faisant réagir un dérivé d'acide éthylcarbamique de formule

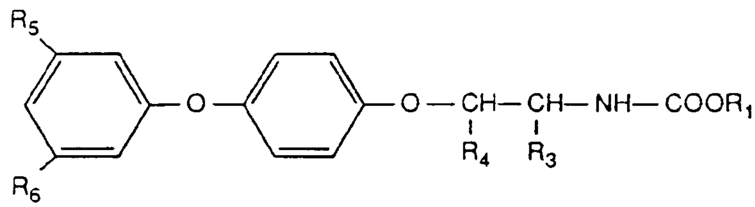

(IV)

dans laquelle $R_1$, $R_3$, $R_4$, $R_5$ et $R_6$ ont les significations données pour la formule I, en présence d'une base avec un halogénure d'acide sulfénique de formule

Hal-S-$R_2$ (V),

dans laquelle $R_2$ a la signification donnée pour la formule I, et Hal représente un halogène, et on oxyde le produit obtenu de formule Ia si c'est souhaité en un composé de formule I dans lequel n représente un ou deux, ou

c) en faisant réagir un dérivé d'acide éthylcarbamique de formule IV en présence d'une base avec un halogénure d'acide sulfinique de formule

Hal-SO-$R_2$ (VI),

dans laquelle $R_2$ a la signification donnée à la formule I et Hal représente un halogène, et on oxyde le produit obtenu de formule

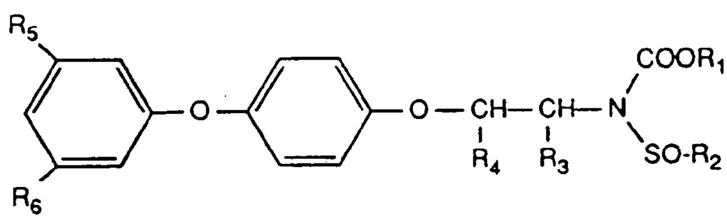

(Ib),

dans laquelle $R_1$, $R_2$, $R_3$, $R_4$, $R_6$ et $R_6$ ont les significations données pour la formule I, le cas échéant pour donner un composé de formule I dans laquelle n représente deux; ou

d) en faisant réagir un dérivé d'acide éthylcarbamique de formule IV en présence-d'une base avec un halogénure d'acide sulfonique de formule

Hal-$SO_2$-$R_2$ (VII),

dans laquelle $R_2$ a la signification donnée à la formule I et Hal représente un halogène, pour donner un composé de formule

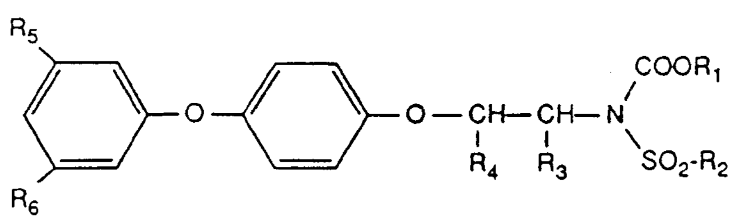

(Ic),

dans laquelle $R_1$, $R_2$, $R_3$, $R_4$, $R_5$ et $R_6$ ont les significations données pour la formule I; ou
e) en acylant un dérivé d'éthylamine de formule

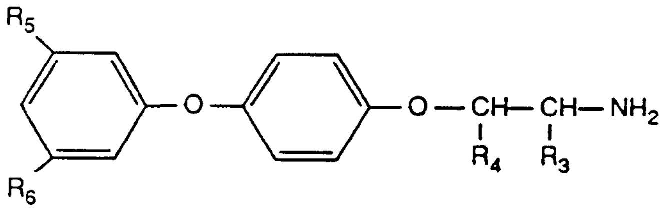

(VIII),

dans laquelle $R_3$, $R_4$, $R_5$ et $R_6$ ont les significations données pour la formule I, en présence d'une base avec un dérivé d'acide halogénoformique de formule

Hal-COOR$_1$ (IX),

dans laquelle $R_1$ a la signification donnée pour la formule I et Hal représente un halogène, et on sulfényle le produit intermédiaire de formule IV en présence d'une base avec un halogénure d'acide sulfénique de formule V et on oxyde le produit obtenu de formule Ia, si c'est souhaité en un composé de formule I, dans lequel n représente un ou deux; ou
f) en sulfénisant un dérivé d'éthylamine de formule VIII en présence d'une base avec un halogénure d'acide sulfénique de formule V, et on acyle le produit intermédiaire obtenu de formule X

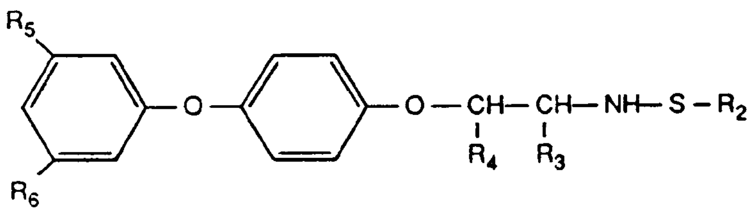

(X),

dans laquelle $R_2$, $R_3$, $R_4$, $R_5$ et $R_5$ ont les significations données pour la formule I, en présence d'une base avec un dérivé d'acide halogénoformique de formule IX et on oxyde le produit obtenu de formule Ia, si c'est souhaité en composé de formule I, dans laquelle n représente un ou deux; ou
g) pour préparer un composé de formule Ic on fait réagir un sel alcalin d'un composé de formule

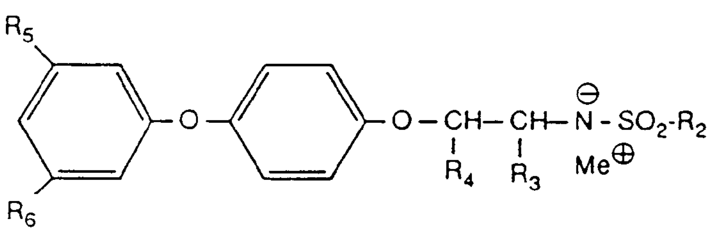

(Xa)

avec un composé de formule IX, $R_1$ à $R_5$ ayant les significations indiquées pour la formule I et $Me^{\oplus}$ est un cation de métal alcalin.

6. Utilisation d'un composé de formule I, selon la revendication 1 pour combattre les parasites des plantes et des animaux, en protection des réserves et en hygiène.

7. Utilisation selon la revendication 6 pour combattre les arthropodes, notamment les insectes et les arachnides.

8. Utilisation selon la revendication 7 pour combattre les larves et les oeufs d'insectes et de papillons nuisibles phytophages.

9. Utilisation selon la revendication 7 pour tuer les oeufs d'insectes et d'ectoparasites endommageant les

plantes.

**10.** Utilisation selon la revendication 7 pour entraver le développement de larves d'insectes et d'ectoparasites endommageant les plantes.